(19) Europäisches Patentamt
European Patent Office
Office européen des brevets

(11) **EP 1 263 420 B1**

(12) **EUROPEAN PATENT SPECIFICATION**

(45) Date of publication and mention
of the grant of the patent:
**07.09.2005 Bulletin 2005/36**

(21) Application number: **01918375.5**

(22) Date of filing: **07.03.2001**

(51) Int Cl.[7]: **A61K 31/07**, A61P 35/00

(86) International application number:
**PCT/US2001/007123**

(87) International publication number:
**WO 2001/066104 (13.09.2001 Gazette 2001/37)**

(54) **RETINOID FORMULATIONS FOR AEROSOLIZATION AND INHALATION**

RETINOID-FORMULIERUNGEN ZUR AEROSOLBILDUNG UND ZUR INHALATION

FORMULATIONS DE RETINOIDES POUR PRODUCTION D'AEROSOL ET INHALATION

(84) Designated Contracting States:
**AT BE CH CY DE DK ES FI FR GB GR IE IT LI LU MC NL PT SE TR**

(30) Priority: **07.03.2000 US 187539 P**

(43) Date of publication of application:
**11.12.2002 Bulletin 2002/50**

(73) Proprietor: **BATTELLE MEMORIAL INSTITUTE Ohio 43201-2693 (US)**

(72) Inventors:
- **DAHL, Alan, R.
  Canal Winchester, OH 43110 (US)**
- **PLACKE, Michael, E.
  Columbus, OH 43212 (US)**
- **ZIMLICH, William, C., Jr.
  Dublin, OH 43017 (US)**
- **DE LUCA, Luigi, M.
  Bethesda, MD 20814 (US)**
- **MULSHINE, James, L.
  Bethesda, MD 20817 (US)**

(74) Representative: **Turi, Michael et al
Samson & Partner
Widenmayerstrasse 5
80538 München (DE)**

(56) References cited:
EP-A- 0 003 064          WO-A-01/10252
WO-A-97/39745          US-A- 5 112 598

- MULSHINE, JAMES L. ET AL: "Regional delivery of retinoids: a new approach to early lung cancer intervention" CLIN. BIOL. BASIS LUNG CANCER PREV. (1998), 273-283. EDITOR(S): MARTINET, YVES. PUBLISHER: BIRKHAEUSER, BASEL, SWITZ. , XP001017973
- MULSHINE J.L.: "Reducing lung cancer risk: Early detection." CHEST, (1999) 116/6 SUPPL. (493S-496S). , XP001018002
- RALEIGH S M ET AL: "Pharmacokinetics of isotretinoin (ISO) in rats following oral dosing or aerosol inhalation." BRITISH JOURNAL OF CANCER, vol. 80, no. SUPPL. 2, July 1999 (1999-07), page 96 XP001012842 Joint Meeting of the British Association for Cancer Research, the British Oncological Association, the Association of Cancer Physicians and the Royal College of Radiologists;Edinburgh, Scotland, UK; July 11-14, 1999 ISSN: 0007-0920
- RALEIGH S M ET AL: "Preclinical evaluation of an isotretinoin (iso) powder formulation for aerosol administration in lung cancer chemoprevention." PROCEEDINGS OF THE AMERICAN ASSOCIATION FOR CANCER RESEARCH ANNUAL, vol. 40, March 1999 (1999-03), page 397 XP001019061 90th Annual Meeting of the American Association for Cancer Research;Philadelphia, Pennsylvania, USA; April 10-14, 1999, March, 1999 ISSN: 0197-016X

## Description

### STATEMENT REGARDING FEDERALLY SPONSORED RESEARCH OR DEVELOPMENT

This invention was made under a Cooperative Research and Development Agreement (CRADA), No. CACR-447, with the National Cancer Institute. The United States of America has rights to this invention as specified in the CRADA.

### BACKGROUND OF THE INVENTION

This invention pertains to retinoid formulations that may be aerosolized and inhaled for the prevention or treatment of diseases of the aerodigestive tract

Lung cancer is the leading cause of cancer death among men and women in the United States, as well as around the world. Since conventional treatments for lung cancer have met with limited success in improving survival outcome, alternative strategies to combat lung cancer have been introduced by various researchers. Oral and intravenous delivery of retinoids, such as 13-*cis* RA, have been investigated in both, animal and human trials. However, retinoid availability to epithelial targets is relatively small, when the retinoid is administered systemically due to retinoid interaction with albumin and /or other protein. It has been reported that 99% of 13-cis RA was present as albumin bound, and that this interaction could not be reversed by competition with high concentrations of retinoid. 13-*cis* RA has shown effectiveness as a chemopreventive agent of oral leukoplakia and head and neck cancer, but with significant toxicity. For the purpose of increasing target tissue bioavailability and reducing general toxicity, inhalation of 13-*cis* RA has been proposed as an alternative chemopreventive approach. Ideally, such an approach would allow delivery of appropriate concentrations of 13-*cis* RA to the pulmonary epithelium, bypassing the marked enterohepatic clearance as well as near universal interaction with albumin, thereby permitting a higher final concentration of active retinoid at the target epithelium.

Former and current smokers, as well as individuals who have been successfully treated for a first aerodigestive cancer, would greatly benefit from drugs that prevent progression of lung neoplasia. This vast group of people comprises half the U.S. adult population, all sharing significant risk for developing lung cancer. Retinoids are necessary for the maintenance of respiratory epithelial cell differentiation *in vivo* and can induce terminal differentiation or apoptosis of initiated epithelial cells and thus have prospects as preventive agents for some forms of cancer. Further, Vitamin A deficiency has been shown to increase the number of benzo(a)pyrene (BaP)-DNA adducts in cultured hamster trachea.

In a randomized clinical trial, the oral administration of isotretinoin (1-2 mg/kg/day) was significantly protective against second aerodigestive tumors in a cohort of previously treated head-and-neck cancer patients. Because of the effectiveness of isotretinoin as a preventative of some forms of cancer, its efficacy as a lung cancer chemopreventive agent is currently under study in several clinical trials (Protocol IDS: UCHSC-92382, NCI-V94-0506 and CBRG-9208, NCI-V92-0159, NBSG-9208).

Enthusiasm for the use of isotretinoin as a chemopreventive agent has been held back, in part due to the occurrence of debilitating drug side effects associated with the doses used in the MD Anderson study, which, based on pharmacokinetic data provided steady-state blood levels of 100-200 ng/ml. Sixteen of the forty-nine patients in this head and neck chemoprevention trial did not complete the course of therapy. Since the benefits of isotretinoin treatment is reduced after cessation of treatment the expectation is that chronic drug administration would be required, making the patient compliance issue critical.

To address the toxicity concerns, investigators have contemplated lowering the dosages of the drug, but it is unclear whether such a change would jeopardize the desired therapeutic effect. Oral doses of I mg/kg failed even to reverse lung metaplasia in smokers. Attempts to lessen the severity of the toxic effects by coadministration of vitamin E are currently under study in clinical trials (Protocol IDS: MDA-DM-97078, NCI-P98-0132), and clearly further work is merited in this critical area.

We reasoned that as lung cancer arises in the lung epithelium, direct application to the target cells would improve the therapeutic index. Aerosol inhalation can deposit drug directly on the population of cells caught up in the early phase of cancer, potentially achieving much more efficiency compared to reliance on diffusion from the blood. There are theoretical bases to expect major differences in potency between oral and inhaled retinoids. Some highly lipophilic compounds can be significantly retarded in their clearance from the lung epithelial surface into the blood stream. As the reverse also is probably the case, the poor results with oral administration may be simply a case of too little drug reaching the target cells in some parts of the lung. In addition, isotretinoin is avidly bound by serum albumin, limiting its availability for promotion of differentiation and inhibition of proliferation. Direct application to the lung epithelium may avoid much of the protein binding, thus greatly increasing potency at the target site.

Surprisingly, despite longstanding interest in isotretinoin, information on its *in vivo* pharmacology as a cancer preventive agent in animal models is scarce. In one study, orally administered isotretinoin of over 300 mg/kg weekly failed to prevent urethane-induced lung cancer in the A/J mouse model (Table 1). Despite this failure, we felt that direct application to the lung epithelium merited evaluated. To test this premise, we elected to expose carcinogen-treated A/J mice to isotretinoin aerosol for this pilot study.

WO 97/39745 A describes the administration of a solution containing among others a retinoid by inhalation to a patient to prevent epithelial cancers of the respiratory tract. The administration of a retinoid with existing neoplasms is not disclosed.

Mulshine, James L. et al: "Regional delivery of retinoids: a new approach to early lung cancer intervention", *Clin. Biol. Basis Lung Cancer Prev.* (1998), 273-283, Editor(s): Martinet, Yves. Publisher: Birkhaeuser, Basel, Switz. hypothesize that the adminstration via inhalation of a retinoid to patients having early cancer may result in a greater therapeutic index compared to oral administration. However, no data for retinoids are presented to support this speculation.

The disclosure in Mulshine, J.L.: "Reducing lung cancer risk: Early detection", *Chest* (1999) 116/6 Suppl. (493S-496S) is similar to that in the above reference Mulshine (1998). Again no experimental work is reported.

Raleigh S.M. et al: "Pharmacokinetics of isotretinoin (ISO) in rats following oral dosing or aerosol inhalation", *British Journal of Cancer*, Vol. 80, no. Suppl. 2 , July 1999 (1999-07), page 96, Joint Meeting of the British Association for Cancer Research and the Royal College of Radiologists; Edinburgh, Scotland, UK; July 11-14, 1999 ISSN: 0007-0920 and Raleigh S.M. et al: "Preclinical evaluation of an isotretinoin (iso) powder formulation for aerosol administration in lung cancer chemoprevention", *Proceedings of the American Association for Cancer Research Annual*, Vol. 40, March 1999 (1999-03), page 397, 90th Annual Meeting of the American Association for Cancer Research; Philadelphia, Pennsylvania, USA, April 10-14, 1999, March 1999; ISSN; 0197-016X have similar dsiclosures. The authors report pharmacokinetic studies regarding the inhalation of a powder isotretinoin formulation by healthy female and male rats compared to the oral administration of that formulation. The determination method of the lung-to-plasma drug ratio, which is higher with the powder aerosol, does not allow an estimate of how much of the powder reached the deep lungs. The treatment of already existing neoplasms is not mentioned.

US 5 112 598 A discloses a pharmaceutical preparation comprising retinoic acid and/or an ester of retinoic acid and/or an ester of retinol in the form of an aerosol for use in topical diseases of mucosal diseases (e.g. diseases of the respiratory epithelium). No data are given as to how much of the aerosol reaches the deep lung. Neoplastic changes and bronchial carcinomas are said to be treatable by the preparation. However, no experimental evidence for these assertions is given.

In EP 0 003 064 A the use of a capsule of a Vitamin A analogue in a cigarette is disclosed. However, upon rupture of the capsule little, if any, active material will reach the deep lungs, since the particles of the active material will fall out of the lighter smoke. The use of the Vitamin A esters for treating neoplasms is not taught by this reference.

WO 01/10252 A which is relevant only under Article 52(3) and (4) EPC discloses the topical administration of a retinoid only in conjunction with a cigarette-type filter device or in a method of creating a lipid peroxidation-reductive deep lung surface by inhaling at least 0.5 mg of high-effect antioxidant and at least 0.1 mg retinoid, both having a particle size of 6 μ or less.

## SUMMARY OF THE INVENTION

Accordingly, these and other disadvantages of the prior art are overcome by this invention which provides the use of retinoic acid or a retinoic acid derivative in the mannfacture of a medicament administered on a chronic basis via inhalation as defined in claim 1.

The present invention demonstrates that 13-*cis* RA, when delivered topically by inhalation, is more effective than when given in the diet to elicit upregulation of key target genes at the target site.

Furthermore, the pulmonary delivery of isotretinoin by inhalation yields evidence of efficacy at weekly pulmonary doses as low as 0.25 mg/kg and suggested efficacy even at 0.04 mg/kg in reducing the pulmonary carcinogenicity of the tobacco carcinogens, NNK and BaP, in A/J mice. Since pulmonary drug delivery deposits drug directly on the tumor compartment, efficacy can be achieved at low doses: mid and low weekly pulmonary doses were < 2% and < 0.3%, respectively, of the highest recommended weekly oral dose of isotretinoin for acne treatment.

Therefore, it is an object of the present invention to provide the use of retinoic acid or a retioic acid derivative in the manufacture of a medicament that may be aerosolized and inhaled by a test subject or patient, wherein efficacy of the retinoid formulation is maximized, and systemic toxicity is minimized.

Further objects, advantages, and novel aspects ofthis invention will become apparent from a consideration of the drawings and subsequent detailed description.

## BRIEF DESCRIPTION OF THE FIGURES

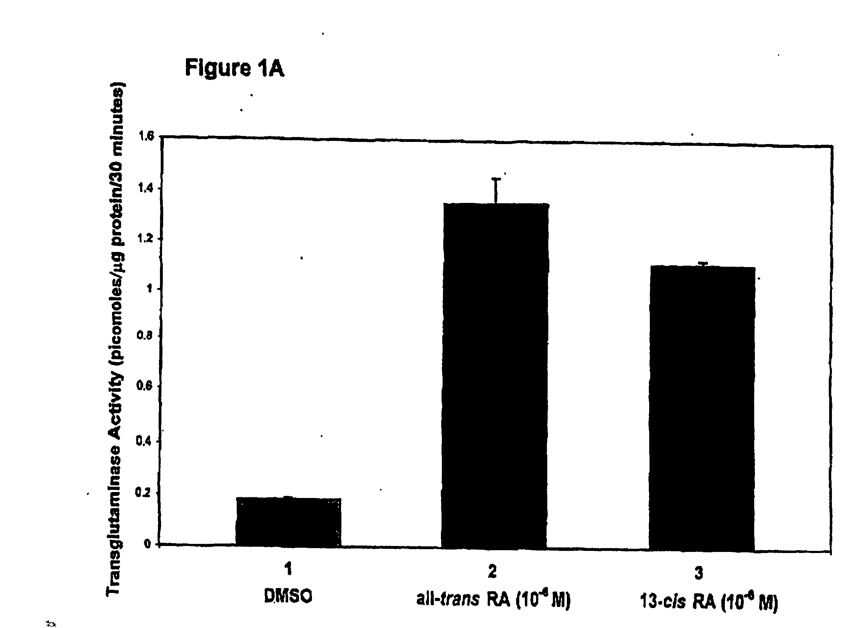

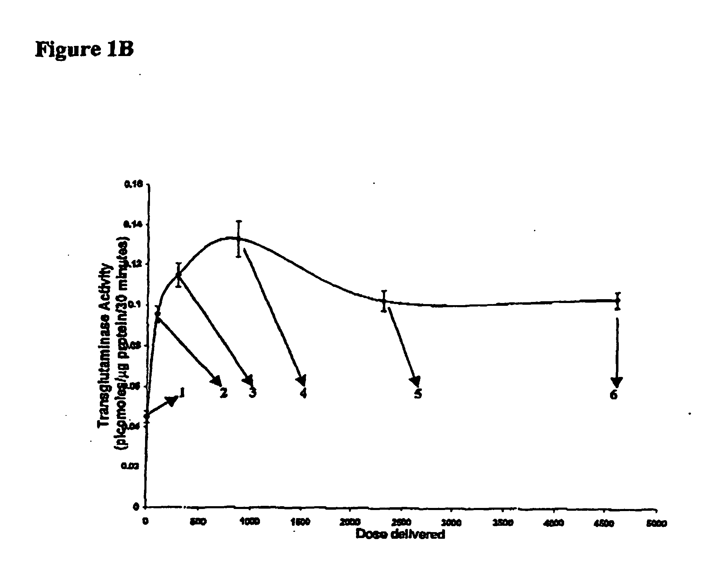

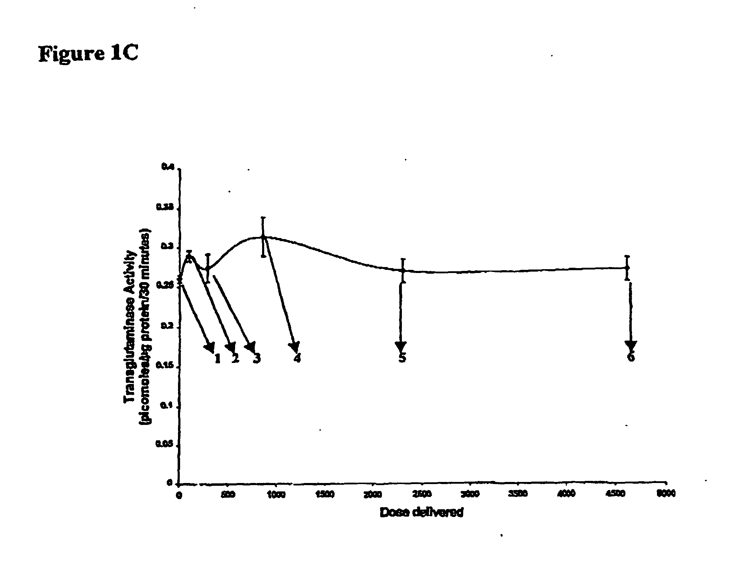

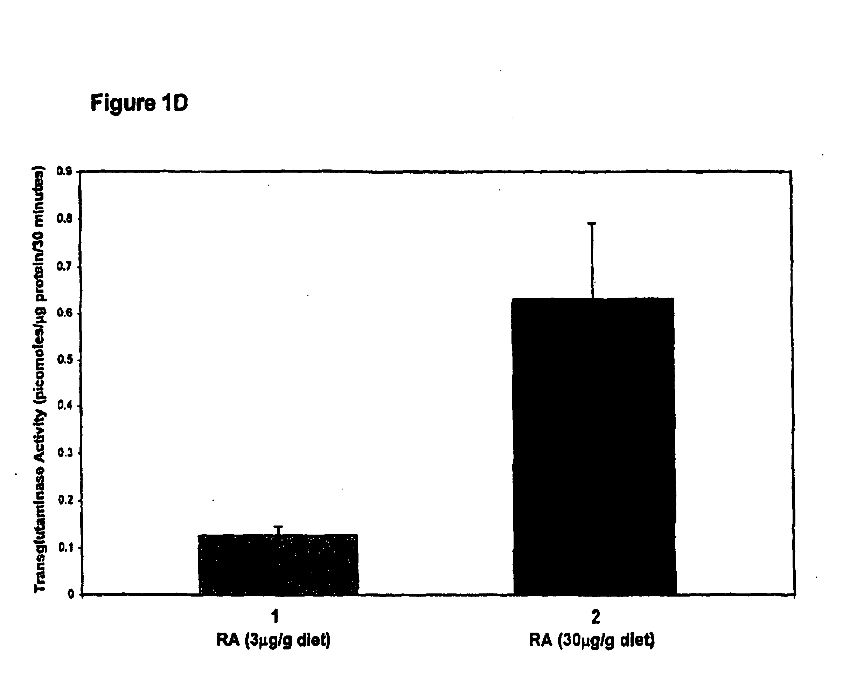

**Figure 1**: Stimulation of TGaseII activity by retinoids.

1A. 13-cis RA and all-trans-RA stimulate TgaseII activity in cultured human breast cancer MCF-7 cells. The average of TGase II activity analysis of three separate dishes +/- standard error for each treatment group. In "1", cells were treated with DMSO for 72 hours (TGase II Activity = 0.183 +/- 0.005 picomoles of putrescine/$\mu$g protein/ 30 minutes); in "2", cells were treated with all-*trans* RA ($10^{-6}$ M) for 72 hours (TGase II Activity = 1.359 +/- 0.098 picomoles/$\mu$g protein/30 minutes) high significance of difference between 1 and 2 (P<0.001); in "3" cells were treated with 13-cis RA ($10^{-6}$ M) for 72 hours (TGase II Activity = 1.118 +/- 0.016 picomoles/$\mu$g protein/30 minutes), high significance of difference between "1" and "3" (P<0.001) without significant difference between "2" and "3" (P<0.07).

**1B.** 13- *cis* RA by inhalation significantly increases TGase II activity (**experiment B**) of rat lung tissue. Four left lungs (one from each rat) were used for each exposure group with 3 measurements per lung (n=12). The mean of the twelve measurements is plotted +/- the standard error. Rats inhaled 13-cis RA aerosol (Table 1). "1" (Vehicle control): lung tissue from rats that inhaled vehicle only (deposited dose = 0) (TGase II Activity = 0.0450 +/- 0.003 picomoles/$\mu$g protein/30 minutes); "2" (Low dose): 39$\mu$g/kg is the total deposited dose of 13- *cis* RA (TGase II Activity = 0.0955 +/- 0.004 picomoles/$\mu$g protein/30 minutes), (P<0.001 between "1" and "2"); "3" (Low-Middle dose): 117$\mu$g/kg is the total deposited dose of 13-cis RA (TGase II Activity = 0.1150 +/- 0.006 picomoles/$\mu$g protein/ 30 minutes), (P<0.001 between "1" and "3") ; "4" (Middle dose): 35 1$\mu$g/kg is the total deposited dose of 13-cis RA (TGase II Activity = 0.1330 +/- 0.009 picomoles/$\mu$g protein/30 minutes), (P<0.001 between "1" and "4"); "5" (Middle-High dose): 936$\mu$g/kg is the total deposited dose of 13-cis RA (TGase II Activity = 0.1020 +/- 0.005 picomoles/$\mu$g protein/30 minutes), (P<0.001 between "1" and "5"); "6" (High dose): 1872$\mu$g/kg is the total deposited dose of 13-cis RA (TGase II Activity = 0.1025 +/- 0.004 picomoles/$\mu$g protein/30 minutes), (P<0.001 between "1" and "6").

1C. Inhaled 13-*cis* RA fails to significantly alter liver TGase II activity (experiment B). Rats inhaled 13-cis RA aerosol (**Table 1**). Measurements were conducted on liver tissue. Methods were the same as for 1B. "1". (Vehicle control): liver tissue from rats that inhaled vehicle (deposited dose = 0) for 240 minutes TGase II Activity = 0.260 +/- 0.005 picomoles/$\mu$g protein/30 minutes; 2 (Low dose): 39$\mu$g/kg is the total deposited dose of 13-cis RA (TGase II Activity = 0.289 +/- 0.007 picomoles/$\mu$g protein/30 minutes), (P<0.285 between "1" and "2"); "3" (Low-Middle dose): 117$\mu$g/kg is the total deposited dose of 13-cis RA (TGase II Activity = 0.273 +/- 0.018 picomoles/$\mu$g protein/ 30 minutes), (P<0.619 between "1" and "3") ; "4" (Middle dose): 351$\mu$g/kg is the total deposited dose of 13-cis RA (TGase II Activity = 0.313 +/- 0.025 picomoles/$\mu$g protein/30 minutes), (P<0.065 between "1" and "4"); "5" (Middle-High dose): 936$\mu$g/kg is the total deposited dose of 13-cis RA (TGase II Activity = 0.269 +/- 0.015 picomoles/$\mu$g protein/30 minutes), (P<0.993 between "1" and "5"); "6" (High dose): 1872$\mu$g/kg is the total deposited dose of 13-cis RA (TGase II Activity = 0.271 +/- 0.015 picomoles/$\mu$g protein/30 minutes, (P<0.758 between "1" and "6").

**1D.** Dietary RA significantly increases mouse liver TGase II activity. Mice were fed RA for 75 weeks at two levels 3 and 30pg/g diet.**(Table 5)** Four different mice from each dietary RA group were used; as for the lungs, mean values of twelve measurements (triplicates for each liver) are plotted +/- the standard error **(Table 6)**. "1": TGaseII activity from the livers of SENCAR mice fed a physiological RA diet (3$\mu$g/g) for 75 weeks (TGase II Activity = 0.125 +/- 0.02 picomoles/$\mu$g protein/30 minutes); "2": TGaseII activity from the livers of SENCAR mice fed a pharmacological RA diet(30$\mu$g/g) for 75 weeks (TGase II Activity = 0.630 +/- 0.16 picomoles/$\mu$g protein/30 minutes), (P<0.003 between "1" and "2").

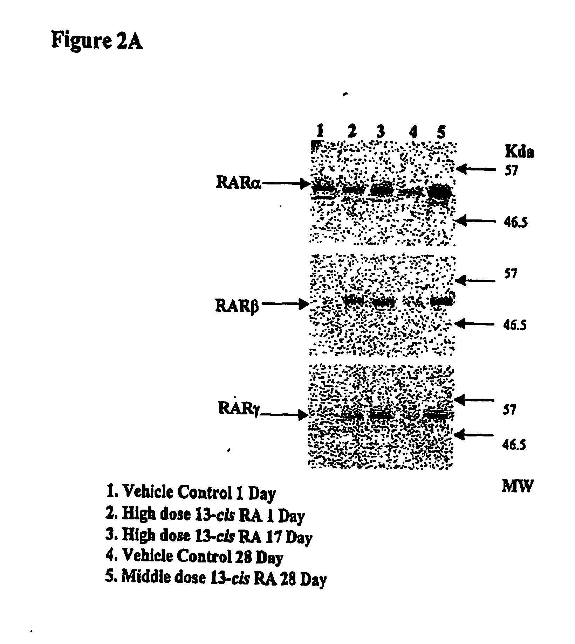

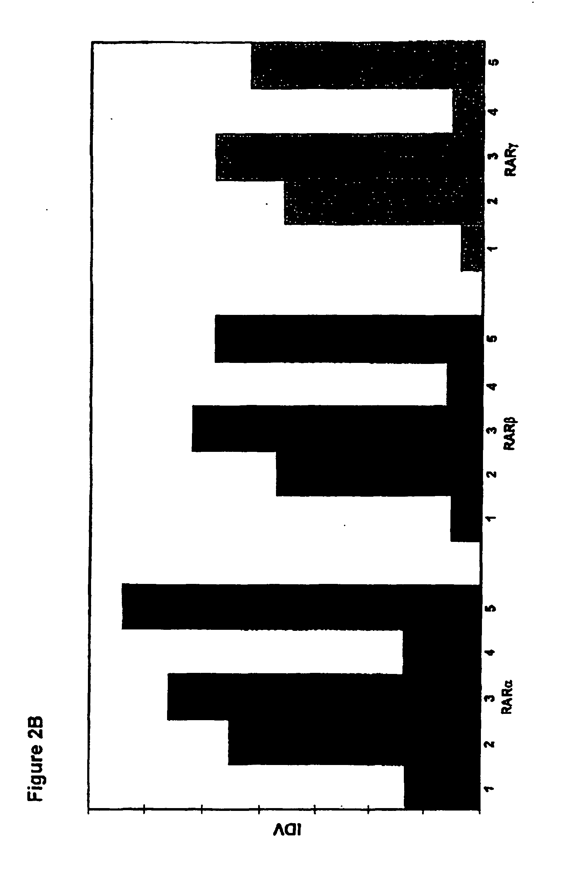

**Figure 2**: Inhaled 13-*cis* RA upregulates rat lung RARs

**2A**. Western blot analysis of rat **(experiment A)** lung samples using polyclonal antibodies to RAR a, $\beta$ and $\gamma$ as explained under Materials and Methods. Rats were exposed to 13-cis-RA by inhalation once daily for two hours **(Table 4)**. Rat lung tissue for lane 1 (Vehicle control 1 Day) samples received vehicle for one day; lane 2 (High dose 13-cis RA, 1 Day) received a calculated total deposited dose of 6.4mg 13-cis RA/kg for one day; lane 3 (High dose 13-cis RA, 17 Day) received a calculated total deposited dose of 6.4mg 13-cis RA/kg for 17 consecutive days; lane 4 (Vehicle control 28 Day) received vehicle for 28 consecutive days; lane 5 (Middle 13-cis RA, 28 Day) received a calculated total deposited dose 1.9mg 13-cis RA/kg for 28 consecutive days.

**2B.** Densitometric analysis of Western blots shown in A. The vertical axis is in arbitrary densitometric units (IDV = Integrated Density Value).

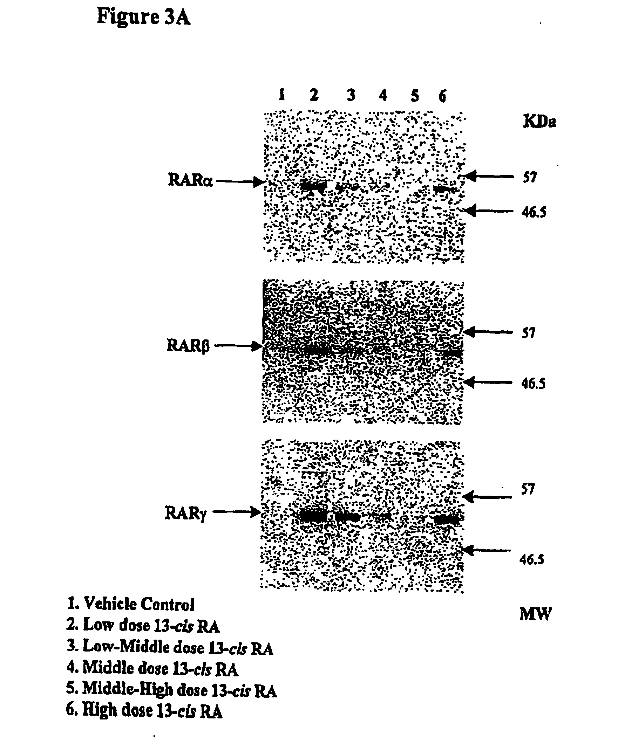

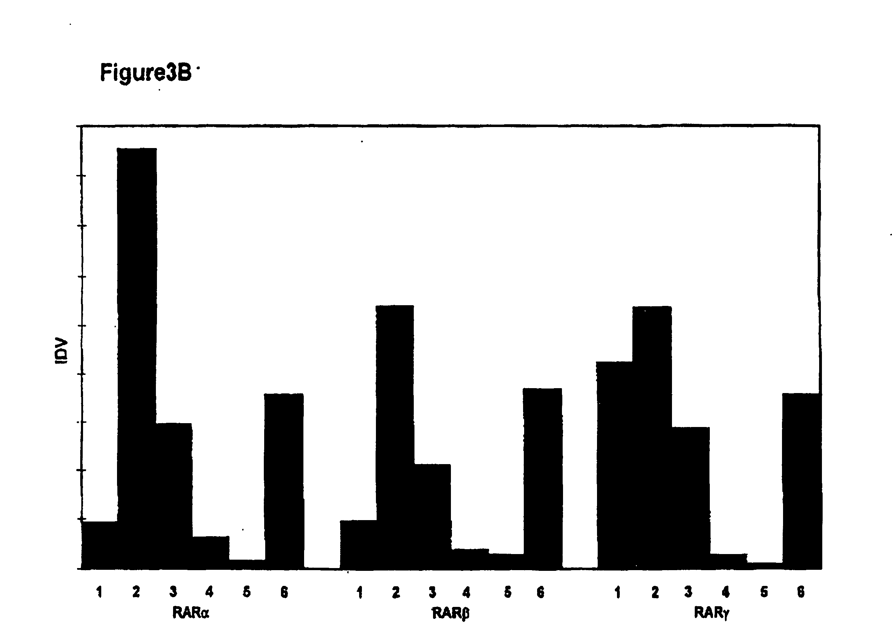

**Figure 3:** Inhaled 13-*cis*-RA upregulates RARs in rat lung tissue at different times of inhalation.

**3A.** Western blot analysis of rat **(experiment B)** lung samples using polyclonal antibodies to RAR $\alpha$, $\beta$ and $\gamma$ as explained under Materials and Methods. Rats inhaled a13-cis RA aerosol **(Table 1)**. Rats lung tissue for lane 1 samples received vehicle for 240minutes; lane 2 (Low dose): 39µg/kg total deposited 13-cis RA; lane 3 (Low-Middle dose):117µg/kg total deposited 13-cis RA; lane 4 (Middle dose): 351 µg/kg total deposited 13-cis RA; lane 5 (Middle-High dose): 936µg/kg total deposited 13-cis RA; lane 6 (High dose): 1872µg/kg total deposited 13-cis RA.

**3B.** Densitometric analysis of Western blots shown in A. The vertical axis is in arbitrary densitometric units (IDV = Integrated Density Value).

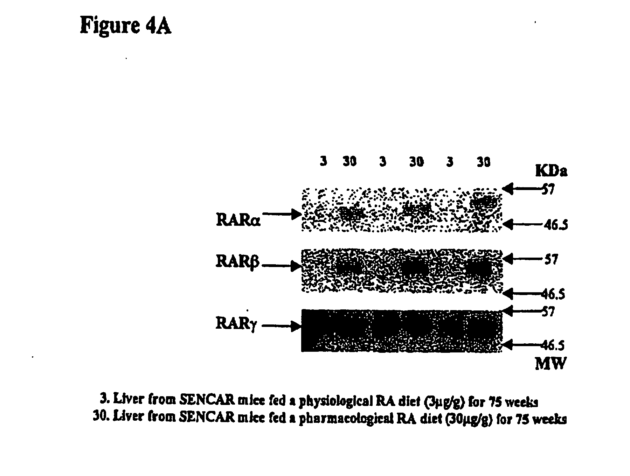

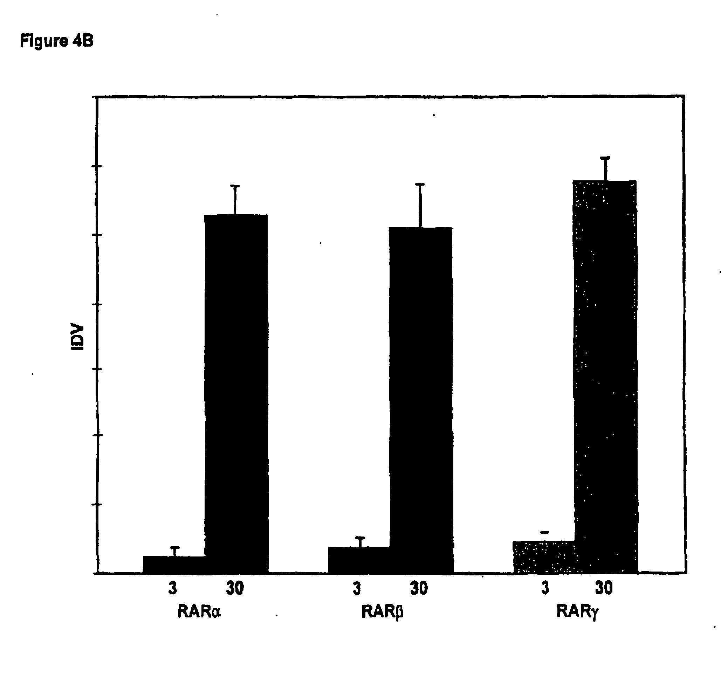

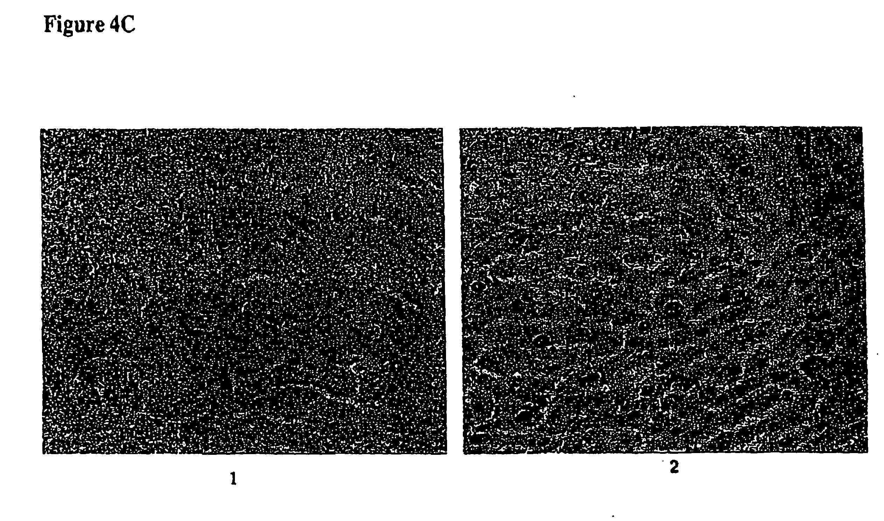

**Figure 4:** Dietary pharmacological RA (30µg/g diet) upregulates RARs in liver from male SENCAR mice

**4A.** Western blot analysis of male SENCAR mouse liver samples using polyclonal antibodies to RAR $\alpha$, $\beta$ and $\gamma$, as explained under Materials and Methods. Mice were fed RA for 75 weeks at two levels, 3 and 30µg/g diet **(Table 5)**. Lane 3: liver tissue from SENCAR mice fed a physiological RA diet(3µg/g) for 75 weeks; lane 30: liver tissue from SENCAR mice fed a pharmacological RA diet(30µg/g) for 75 weeks.

**4B.** Average of the densitometric analysis of three different Western blots shown in A. The vertical axis is in arbitrary densitometric units( IDV = Integrated Density Value). RARa 12.1 +/- 7.2 (3µg/g) compared with 264 +/- 21.3 (30µg/g) (P<0.0001); RAR$\beta$ 18.9 +/- 7.4 (3µg/g) compared with 254 +/- 31.9 (30µg/g) (P<0.0002); RAR$\gamma$ 23.1 +/- 6.7 (3pg/g) compared with 288 +/- 17.4 (30µg/g) (P<0.0001).

**4C.** Immunohistochemical analysis of male SENCAR mouse liver samples using polyclonal antibody to RAR a as explained under Materials and Methods.

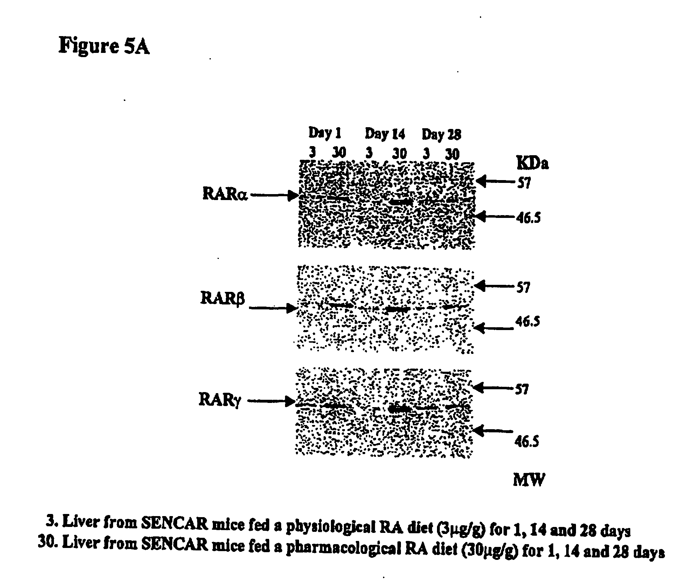

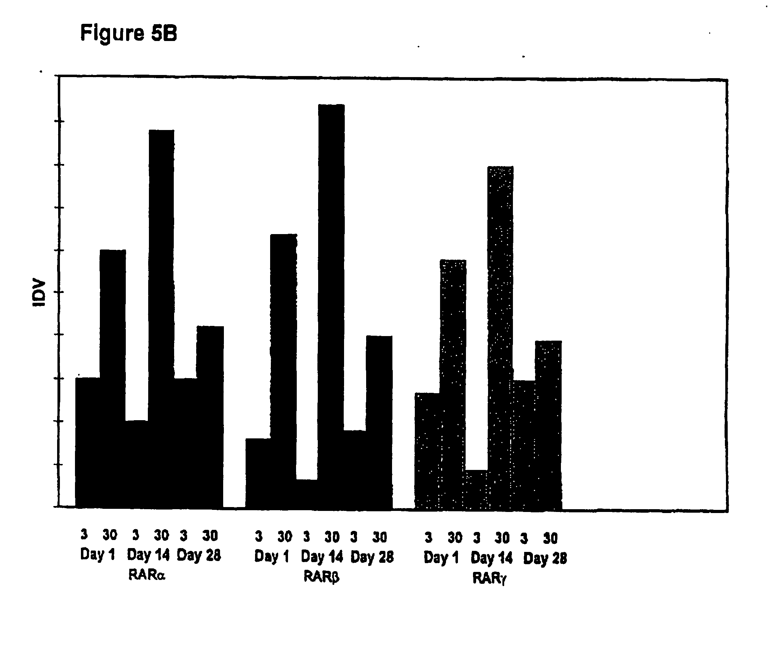

**Figure 5:** Pharmacological dietary RA (30µg/g diet) upregulates RARs in liver from SENCAR mice

**5A.** Western blot analysis of SENCAR mouse liver samples using polyclonal antibodies to RAR $\alpha$, $\beta$ and $\gamma$, as explained under Materials and Methods. Mice were fed RA for different time at two levels, 3 and 30µg/g diet (**Table 7).** Lane 3: liver from SENCAR mice fed a physiological RA diet(3µg/g) for 1,14 and 28 days; lane 30: liver from SENCAR mice fed a pharmacological RA diet(30µg/g) for 1, 14 and 28 days.

**5B**. Densitometric analysis of Western blots shown in A. The vertical axis is in arbitrary densitometric units (IDV = Integrated Density Value).

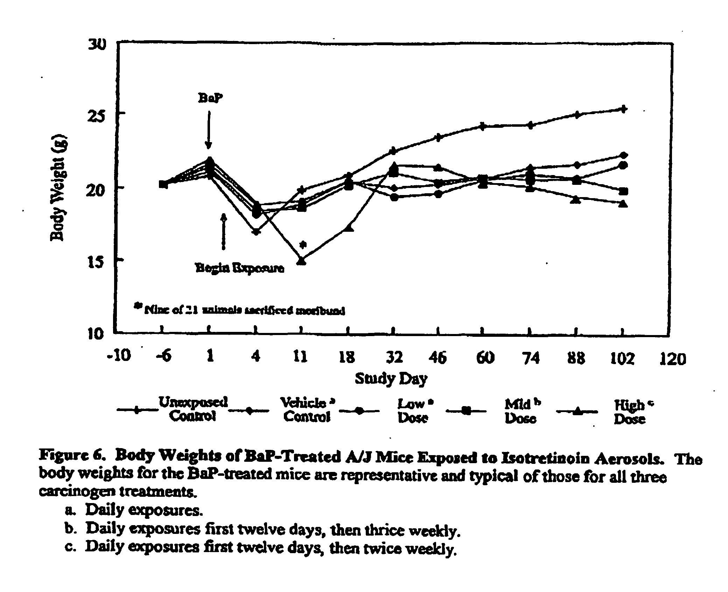

**Figure 6. Body Weights of BaP-Treated A/J Mice Exposed to Isotretinoin Aerosols.** The body weights for the BaP-treated mice are representative and typical of those for all three carcinogen treatments.

a. Daily exposures.
b. Daily exposures first twelve days, then thrice weekly.
c. Daily exposures first twelve days, then twice weekly.

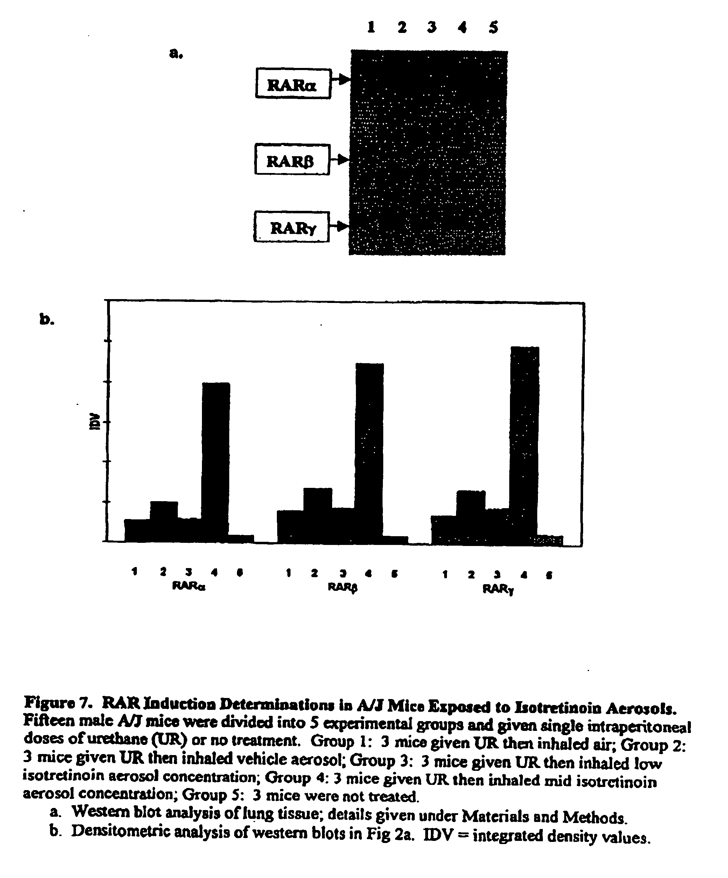

**Figure 7. RAR Induction Determinations in A/J Mice Exposed to Isotretinoin Aerosols.** Fifteen male A/J mice were divided into 5 experimental groups and given single intraperitoneal doses of urethane (UR) or no treatment. Group 1: 3 mice given UR then inhaled air; Group 2: 3 mice given UR then inhaled vehicle aerosol; Group 3: 3 mice given UR then inhaled low isotretinoin aerosol concentration; Group 4: 3 mice given UR then inhaled mid isotretinoin aerosol concentration; Group 5: 3 mice were not treated.

a. Western blot analysis of lung tissue; details given under Materials and Methods.
b. Densitometric analysis of western blots in Fig 2a. IDV = integrated

Reference will now be made in detail to the present preferred embodiment to the invention, examples of which are illustrated in the accompanying figures.

## DETAILED DESCRIPTION

The present invention is directed to the use of retinoic acid or a retinoic acid derivative as a chemoprotectant in the manufacture of a medicament administered on a chronic basis via inhalation as defined in claim 1.

The use of the invention contemplates that a patient may be treated for lung cancer using conventional treatment methods prior to initiation of chronic treatment by inhalation of an aerosol of a retinoic acid derivative. Standard treatment methods are within the skill of the art and include for example, surgical removal of diseased lung tissue, chemotherapy with an anti-cancer drug or radiation treatment. A combination of such treatments, e.g., surgery and radiation may also be employed.

As is understood by one skilled in the art the term "chronic" is used to mean the administration of the active drug substance i.e., the retinoic acid derivatives disclosed herein for a prolonged period of time, perhaps for the life of the patient, usually on a daily basis or several times per week. The active drug substance described herein will generally be self-administered by the patient via inhalation of an aerosol generated using commercially available aerosol generating devices such as a nebulizer, pump atomizer, metered dose inhaler, or electrohydrodynamic aerosol generating device as described below.

The term "active drug substance" or "retinoic acid derivative" as used in the use of the invention means retinoic acid as well as other natural and synthetic retinoids and structurally related compounds; and all compounds interacting with retinoic acid receptors (RARs) or retinoid X receptors (RXRs) such as: 9-cis retinoic acid (9-cis RA), 13-cis RA, all trans RA, retinal, retinol, fenretinide, etretinate, retinyl palmitate, 11-cis retinoic acid, CRBP-retinal, 9, 13, and 11-cis retinal, CH 55, AM 80, retinyl acetate, beta carotene, heterocarotinoids, acitretin, tazarotene.

The Retinoic acid derivatives used in the use disclosed herein will be administered to the patient at an "amount effective to prevent neoplasias" from progressing to lung cancer. Generally, the active drug substance will be administered by inhalation of an aerosol at from about 0.03 to about 0.17 ng/cm$^2$ of lung surface area and preferably from about 0.03 to about 0.05 ng/cm$^2$ lung surface area. Alternatively, as would be recognized by one skilled in the art, dosage of active drug substance may be calculated using the estimated weight of the lung tissue of the patient. Dosage of active drug substance calculated on this basis will generally range from about 0.84 to about 230 µg/g of human lung tissue. Another embodiment of the invention contemplates an active drug substance in the form of a dry powder. The dry powder may be administered by any of several commercially available dry powder inhalers.

An object of the present invention is to permit direct action of the retinoic acid derivative on the respiratory epithelium, the epithelium of the nose and throat cavity and preferably the epithelia of the trachea and deep bronchial tract; all collectively referred to herein as the "aerodigestive tract". Diseases of the aerodigestive tract include epithelial diseases of the nose and throat cavity,and lung and airways such as emphysema, chronic obstructive pulmonary disease COPD), neoplasms, dysplasia, metaplasia, inflammation, hyperplasia. As used herein the term "preneoplasm" or "preneoplasia" is used to refer to collectively: the conditions of dysplasia, metaplasia, inflammation, and hyperplasia of the epithelia of the aerodigestive tract.

Preparation of aerosol formulations of a retinoic acid derivative described herein is within the skill of the art; for example see Inhalation *Aerosols: Physical and Biological Basis for Therapy, Lung Biology in Health and Disease Series.* Edited by A.J. Hickey, Marcel Dekker (1996) *and Pharmaceutical Inhalation Aerosol Technology.* Edited by A. J. Hickey, Marcel Dekker, NY, (1992). An aerosol formulation containing a retinoic acid derivative described herein as the active drug substance may additionally contain one or more inactive excipients such as carriers (e.g., inert gases, liquids or powders), antioxidants, and the like.

Illustrative of the carriers that may be used herein are pharmacologically acceptable organic solvents (such as: ethanol, benzyl alcohol, glycerin, glycolfurol, isopropyl alcohol, polyethylene glycol, propylene glycol, triacetin isopropyl myristate, isopropyl palmitate); antioxidants (such as: tocopherol, ascorbic acid, ascorbyl palmitate, butylated hydroxyanisole, butylated hydroxytoluene, fumaric acid, malic acid, propyl gallate, ascorbate, bisulfite, Trolox, tocopheryl acetate, acetyl cysteine, phosphatidyl choline) emulsifiers (such as: cetostearyl alcohol, glycerol monostearate, lanolin, lanolin alcohols, oleic acid, polyoxyethylene alkyl ethers and esters, propylene glycol esters, detergents).

It has been found that topical delivery of the active drug substance by inhalation is more effective than when the retinoic acid derivative is given in the diet to elicit up-regulation of key target genes in the epithelial tissue in the lung. Inhaled 13-*cis* retinoic acids increased lung TGase II activity without a significant effect on liver enzyme activity, whereas dietary retinoic acid had a significant effect on liver TGase II activity.

## I. Topical Delivery of 13-cis Retinoic Acid by Inhalation Upregulates Expression of Rodent Lung but Not Liver Retinoic Acid Receptors

As a preclinical study to the present invention, normal rats were exposed to inhaled concentrations of 13-*cis* RA, specific biomarkers were examined to monitor effect. TGase II and the RARs were chosen as biomarkers because they are first order dependency genes, i.e. they have been shown to contain a RA-responsive element in their promoter.

MCF-7 cells were seeded at a density of 1.5 x 10$^5$ cells/ml medium (500ml DMEM + 56.2ml FBS + 5.6m1 AB/AM) in 6cm diameter dishes for 24 hours and treated with either DMSO, RA, or 13-*cis* RA at 10$^{-6}$ M and grown to confluence (about 72 hours). Cells were harvested and Transglutaminase II activity was measured as described below. Nebulizer solution, inhalation procedures, details of animal housing are described below.

### Rat inhalation, Experiment A

Rats (n=97) (Sprague-Dawley from Charles River) were divided into five experimental groups and were given varying amounts of 13-*cis* RA by inhalation. The experimental groups were as follows (**Table 4**) :1.Vehicle (10/9 mix of PEG 300 and Ethanol with 0.5% of ascorbic acid and 0.5% phosphatidyl choline) control, day 1: rats (19) inhaled vehicle for 2 hr for one day ; 2. High dose 13-*cis* RA, day 1: rats (22) inhaled a solution containing 13-*cis*-RA at a concentration of 104µg/liter (projected daily inhaled dose 10mg/kg body weight) for 2 hr for one day; 3. High dose 13-*cis* RA, day 17: rats (22) inhaled the same solution as in 2 for 2 hr each day for 17 days; 4. Vehicle control, day 28:

rats (16) inhaled vehicle as for group 1 for a period of 28 days; 5. Middle dose 13-*cis* RA, day 28: rats (18) inhaled a solution containing 13-*cis*-RA at a concentration of 31μg/liter (projected daily inhaled dose 3mg/kg body weight) for 2 hr for each of 28 days.

### Rat inhalation, Experiment B

Male rats (n=23) (Sprague-Dawley from Charles River) were divided into six experimental groups and were given varying amounts of 13-*cis* RA through an inhalant apparatus once daily for different times each day for 14 days. The experimental groups were as follows (**Table 1**) :1.Vehicle control : rats (3) inhaled vehicle for 240 minutes; 2. Low dose 13-*cis* RA: rats (4) inhaled a solution containing 13-*cis* RA at a concentration of 62.2μg/liter (inhaled dose 115.0μg/kg body weight) in 5 minutes; 3. Low-Middle dose 13-*cis* RA : rats (4) inhaled a solution containing 13-*cis* RA at a concentration of 62.2μg/liter (inhaled dose 334.4μg/kg body weight) in 15 minutes; 4.Middle dose 13-*cis* RA: rats (4) inhaled a solution containing *13-cis* RA at a concentration of 62.2μg/liter (inhaled dose 1012.3μg/kg body weight) in 45 minutes; 5. Middle-High dose 13-*cis* RA : rats (4) inhaled a solution containing 13-*cis* RA at a concentration of 62.2μg/liter (inhaled dose 2843.4μg/kg body weight) in 120 minutes; 6. High dose 13-*cis* RA : rats (4) inhaled a solution containing 13-*cis* RA at a concentration of 62.2μg/liter (inhaled dose 5935.6μg/kg body weight) in 240 minutes.

### Dietary RA studies in SENCAR mice

Male SENCAR mice (10) were divided into two experimental groups and were fed varying amounts of RA in the diet. The experimental groups were divided in two groups of five mice (**Table 5)** each including a low and high dose group that were fed either a physiological RA diet of 3μg/g diet) or a pharmacological RA diet (30μg/g diet) for 75 weeks, respectively.

### Immunohistochemical staining

Liver tissue (approximately 300mg) was fixed in 10% formalin, embedded in paraffin and 5μm sections were used for immunohistochemistry. Staining for RAR was similar to our previously described protocol {8656}. ABC kit Mouse/Rabbit IgG and DAB Substrate kit were used (Vector Laboratories Inc., 30 Ingold Road Burlingame, CA).

### Time course of dietary RA effect on RARs

SENCAR mice (30) were divided into six experimental groups. The experimental groups were as follows **(Table 7):** groups 1, 3 and 5 (5 mice each) were fed a physiological RA diet (3μg/g diet) for 1, 14 and 28 days; groups 2, 4 and 6 (each of 5 mice) were fed a pharmacological RA diet (30μg/g diet) for 1, 14 and 28 days.

### Antibodies

Polyclonal rabbit anti-mouse antibodies against RAR, and (SANTA CRUZ Biotechnology Inc., San Francisco, CA) were used. BM Chemiluminescense Western Blotting Kit (Mouse/Rabbit) was used (Boehringer Mannheim Corporation, Indianapolis) for the Westerns.

### Apparatus and reagents for Western blot analysis

X Cell II Mini-Cell & Blot Module was employed with 10% Tris-Glycine Gels and Transfer Buffer; Tris-Glycine SDS Sample Buffer; Tris-Glycine SDS was used as Running Buffer (NOVEX - NOVEL Experimental Technology Inc., San Francisco, CA).

### TGase II assay

Cultured cells were placed in 100 μl scraping buffer [A: 2800μl (400μl 0.5M Na-Phosphate, 500μl 0.01M EDTA, 100μl 1M DTT , 9ml PBS total 10ml) + B:700μl (10μl 20mg/ml PMSF 790μl PBS total 800μl)] for each dish. Cells were broken by a sonicator and kept in ice until used. TGase II assay was conducted as described below and previously {8217}.

For liver tissue, a piece of approximately 100-400mg was used. Tissue was diced in small pieces and homogenized in approximately 2 volumes of scraping buffer for 2-3 minutes at 4°C. Samples were centrifuged at 14,000xg for 30 minutes at 4°C. The supernatant was removed and kept in ice until used.

Enzyme assay mixtures were prepared by adding 80μl of SUBSTRATE mixture (322.8μl $H_2O$, 120μl 0.5M

NaBorate, 60μl 0.01M EDTA, 60μl 0.1M CaCl$_2$, 6μl 1M DTT, 240μl dimethyl casein, 30μl Triton X-100, 1.2μl [$^3$H]-Putrescine, 120μl Putrescine) to 20μl sample, 20μl scraping buffer for blank control , 20 μl (18μl scraping buffer + 2μl TGase II) for positive control. Samples were mixed in 15ml tubes, the tubes were shaken before putting them into a 28°C water bath for 30 minutes. Reactions were slowed down in an ice bath. 80μl of the incubation mixture was adsorbed onto paper disks. These were immediately dropped into cold 10% TCA (0.1% Putrescine) and washed with agitation for 7 minutes, followed by 2 additional washes with 5% TCA (0.05% putrescine) for 5~7 minutes, and once more with cold (-20°C) 95% ethanol for 5 minutes. Paper disks were dried and placed into 5ml Aquasol, and counted in a Liquid Scintillation Counter. Protein concentration determination was conducted by the Bradford method.

### Western blot analysis

### Sample preparation

Tissues were collected, frozen in dry ice and kept at -70°C until used. 500mg aliquots were diced in small pieces and homogenized in 300μl of cold PBS, using the hand held homogenizer for 2~3 minutes. Sample and washes were centrifuged at 5000xg for 20 minutes. The pellet was suspended in 400 μl of ice-cold Buffer A [2μl 0.5M EDTA, 10μl 100mM EGTA, 50μl 100mM PMSF, 10μl 1M DTT, 10ml (10mM HEPES pH 7.9 + 10mM KCl)], and left at ice temperature for 15 minutes. 25 μl of 10% solution of NP-40 was added and samples were mixed in a vortex vigorously for 10 seconds. Samples were centrifuged at 14,000xg for 1 minute at 4°C and the pellet treated once more in this fashion (resuspended etc.). The supernatant was removed and 100μl of ice-cold Buffer C [4μl 0.5M EDTA, 20μl 100mM EGTA, 20μl 100mM PMSF, 2μl 1 M DTT, 2ml (20mM HEPES pH 7.9 + 0.4M NaCl)] was added. Pellets were resuspended by tapping gently on the bottom of the Eppendorf tubes. Samples were rocked vigorously in a bucket of ice on an orbital shaker for 30 minutes. Samples were centrifuged at 14,000 xg for 5 minutes at 4°C. Protein determination of supernatant was conducted by the Bradford method.

### Sample analysis

Samples were prepared by adding one part of the Sample Buffer to one part of the sample and mixing well. Samples were heated at 95°C for 5 minutes to induce denaturation. 5μl of Rainbow Standard and 5μl ofBiotinylated Molecular Marker were added to parallel wells. 20μg protein per sample was loaded in each lane. Electrophoresis was performed with voltage set at 125V for about 1 ~ 1.5 hours. Gel transfer was executed at 25V for 2 hours. The membrane was stained in Ponceau S. for 5 minutes and destained with one wash of 5% acetic acid. The membrane was then washed in TBS solution until the staining disappeared. Membranes were incubated in 1ml blocking solution and 9ml TBS for 60 minutes. Membranes were incubated in 1ml of blocking solution and 19ml TBS along with 20μl primary antibody solution overnight (dilution of 1 : 1000). Membranes were washed in PBS-Tween 20, 3 times for 10 minutes each. They were finally incubated in 1ml blocking solution and 19ml TBS along with 20μl Antibiotin HRP - Linked Antibody and 2μl of secondary (rabbit anti-mouse antibody) (1 : 10,000 dilution) for 30 minutes. The membranes were washed in PBS - Tween 20, 4 times for 10 minutes each. The film was exposed for development and detection.

### *Demonstration that 13-cis RA stimulates TGase II activity and comparison with all-trans RA in cultured human breast cancer MCF-7 cells*

Prior to using 13-*cis* RA by the inhalation route, its ability to upregulate the retinoid responsive and possible biomarker TGase II, compared to RA was tested. The details of this experiment are shown in **Fig.1** A shows that 13-*cis* RA (6.1 Fold) is nearly as effective as RA (7.4 Fold) in stimulating TGase II activity in cultured human breast cancer MCF-7 cells.

### *Inhaled 13-cis RA stimulates TGase II activity in rat lung, but not liver tissue*

The details of this experiment are shown in **Table 1, Table 2** and **Fig. 1B** show a significant (2.9 Fold) stimulation by inhaled 13-*cis* RA of lung TGase II activity. The increase was evident with a dose as low as 69μg/kg given daily for 14 days and reached a maximum at a inhaled dose of 1012.3μg/kg, i.e. after 45 minutes of inhalation of the aerosol. It then decreased down to 1.2 fold with larger amounts of inhaled retinoid. **Table 3** and **Fig. 1C** show no significant effect of inhaled 13-*cis* RA on liver TGase II activity with up to 5.93mg/kg of inhaled dose. Therefore, the inhalation route appeared to yield an immediate and sustained effect of the retinoid on TGase II activity.

*Dietary RA stimulates TGase II activity in SENCAR mouse liver*

The details of this experiment are shown in **Table 5** and **Table 6**. We tested the hypothesis that dietary RA might be effective in stimulating TGase II activity in SENCAR mouse liver tissue. We used SENCAR mice fed either a physiological RA diet (3μg/g diet) or a pharmacological RA diet (30μg/g diet) for 75 weeks as indicated in **Table 5. Fig. 1D** shows that dietary RA (30μg/g diet) is effective in stimulating TGase II activity in liver from male SENCAR mouse by 5.0 fold over physiological RA (3μg/g diet).

*Inhaled 13-cis RA stimulates RAR proteins in rat lung, but not liver tissue*

This experiment was conducted to study the specific effect of inhaled 13-*cis* RA on lung tissue of the rat. The details of this experiment are shown in **Table 4**. Inhalation exposure to 13-*cis* RA **(Fig. 2 A)** at high (lanes 2 and 3) or middle (lane 5) doses as specified in **Fig. 2** legend caused an increase of between 3.4 and 4.7 fold over solvent control (lanes 1 and 4) at different times of daily exposures to the retinoid for RAR ; an increase of between 7.2 and 10 fold for RAR ; and between 8.1 and 12.9 fold for RAR (**Fig. 2 B**). Therefore, RARs appear to be highly responsive to inhaled *13-cis* RA in the rat lung tissue when inhaled.

Next, the effect of inhaled 13-*cis* RA had any effect on liver RARs was studied. Western blot analysis of rat liver samples from the same rats as shown in **Fig 2A** failed to show any increase in RARs after administration of 13-*cis* RA by inhalation (not shown), supporting the concept that topical administration is an effective means of local biomarker enhancement, but the systemic concentration of 13-cis RA that results from inhaled drug delivery is insufficient to induce liver RARs.

Further, rats were made to inhale different amounts of the same solution of 13-*cis* RA, by varying the exposure time between 5 and 240 minutes resulting in different inhaled doses between 115.0 and 5935.6μg/ kg body weight every day for 14 consecutive days, **Table 1** Western blot analysis of these rat lung tissues is shown in **Fig. 3 A** and its densitometry in **Figure 3 B**. As for the previous experiment, inhaled 13-*cis* RA effectively increased the amount of RAR proteins between 1.2 and 38.8 fold for RAR, 1.6 and 30.6 for RAR and 2.2 and 74.0 for RAR **(Fig. 3 B)**. However, there was no obvious dose-response relationship and it appeared that the most effective exposure was the shortest one (i. e. for 5 min. at 115.0μg/kg body weight). In contrast to the observed stimulation for lung RARs, liver RARs were not responsive to inhaled *13-cis* RA (not shown).

*Dietary RA increases liver RARs*

Next, the hypothesis that dietary RA might be effective in increasing liver RARs was tested. We used SENCAR mice fed either a physiological RA diet (3μg/g diet) or a pharmacological RA diet (30μg/g diet) for 75 weeks as indicated in **Table 5.** Dietary RA (30μg/g diet) upregulated RARs (**Fig. 4 A**) in liver from male SENCAR mice by 21.8 fold for RAR , 13.5 fold for RAR and 12.5 fold for RAR (**Fig. 4 B**).

**Fig. 4 C** shows a representative immunohistochemical analysis of male SENCAR mouse liver samples using polyclonal antibody to RARα as explained under Materials and Methods. A marked increase in staining was observed in the nuclei of mice consuming the pharmacological RA diet compared to physiological RA.

The ability of dietary RA to increase RARs at shorter times of dietary consumption of physiological and pharmacological levels of RA, as indicated in **Table 7. Fig. 5 A** and **B** show an induction of liver RAR between 1.4 and 4.4 fold; between 2.2 and 14.3 fold for RAR and between 1.3 and 8.9 fold for RAR. In sharp contrast, no effect of dietary RA was observed on lung tissue RARs (not shown).

*Discussion of Upregulation Inhalation Experiments*

Retinoids are key regulators of lung epithelial cell differentiation and act as ligands of the nuclear receptors RARs. They have been utilized in chemoprevention approaches in different tissues and 13-*cis* RA has been shown to be effective against leukoplakia as well as against head and neck cancer. However, systemic administration presents with considerable problems if one takes into account the interactive nature of the retinoid molecules and the high affinity of albumin for retinoids in the blood. In fact, we have previously shown that the uptake of serum retinoids in cultured cells is inversely related to the concentration of albumin in the culture medium. The high affinity interaction of retinoids with albumin and possibly other proteins may limit attainment of effective concentrations of retinoid in lung epithelium and impede chemopreventive activity. Therefore, we have suggested an alternative approach i.e. the possibility that topical delivery to the lung by inhalation may permit more efficacious chemopreventive approaches.

With the type of efficient delivery system described below, the amount of drug that is required to achieve critical retinoid dose concentration in bronchial epithelium is a small fraction of the doses that have been used clinically. Since only a small amount of drug would be administered per dose, both, potential for side effects and the cost economy of the drug should be improved compared to the standard oral drug delivery approach.

The present invention has tested the hypothesis that 13-*cis* RA, when delivered topically by inhalation, may be more effective than when given in the diet to elicit upregulation of key target genes at the target site. Our experiments are consistent with this hypothesis. Inhaled 13-*cis* RA increased lung TGase II activity (P<0.001) without significant effect on liver enzyme activity(P< 0.544), but fed RA has a significant effect on liver enzyme activity of SENCAR mice (P<0.003). Further, inhaled 13-*cis* RA greatly stimulated pulmonary RAR expression at the protein level for all three receptors, while it failed to have any significant effect on liver RARs. Interestingly a marked stimulation of RARs was already observed at five minute of inhalation (Fig 3A). The stimulation of RARα, β and γ in the lung samples confirms that the aerosol apparatus effectively delivered 13-*cis* RA to the lungs and therefore permitted the immediate response in biomarker upregulation.

### Summary of Inhalation Upregulation Experiments

The present invention tested the hypothesis that chemopreventive retinoids, such as 13-*cis* retinoic acid (13-*cis* RA), may be more effective if delivered to the lung epithelium by inhalation, rather than given in the diet. We used the enzyme transglutaminase II (TGase II) and retinoic acid receptors as possible biomarkers of retinoid activity. First we verified that 13-*cis* RA was comparable to all-*trans*-retinoic acid (RA) in its ability to induce the target gene TGase II in cultured human cells. Next, we used 13-*cis* RA, a compound with lesser toxicity than RA, for our inhalation studies. Inhaled 13-*cis* RA had a significant stimulatory activity on TGase II in lung (P<0.001), but not in liver tissue (P<0.544). Further, RAR, and proteins were found to be highly sensitive biomarkers of retinoid exposure. Inhaled 13-*cis* RA (at daily deposited dose of 6.4mg/kg/day) was effective in upregulating the expression of lung tissue RAR , and at day 1 (RAR by 3.4 fold; RAR by 7.2 and RAR by 9.7 fold), and at day 17 (RAR by 4.2 fold; RAR by 10.0 and RAR by 12.9 fold). At daily deposited dose of 1.9mg/kg/day was also effective, but required more exposures. At day 28 of exposure, lung RAR was induced by 4.7 fold; RAR by 8.0 and RAR by 8.1 fold. Inhalation of the same aerosol concentration in graded exposures, for durations from 5 to 240 min daily, for 14 days induced all RARs from 30.6 to 74 fold at the shortest exposure time. By contrast, long term feeding of a diet containing pharmacological RA (30μg/g diet) failed to induce RARs of SENCAR mouse lung tissue, though it markedly induced liver RARs (RAR by 21.8 fold; RAR by 13.5 and RAR by 12.5 fold). A striking increase of RAR expression was evident in the nuclei of hepatocytes from these mice. A time-course study revealed that pharmacological dietary RA stimulated RAR , and already at day 1 by 2, 4, and 2.1 fold respectively over physiological RA (3μg/g diet) without any measurable effect on lung tissue RARs. These data demonstrate that 13-*cis* RA delivered to the lung tissue of rats is a potent stimulant of lung RARs, but has no effect on liver RARs. Conversely, dietary RA stimulates liver RARs, but fails to affect lung tissue RARs. These data together with the data on TGase II support the concept that epithelial delivery of chemopreventive retinoids to lung tissue may be a more efficacious way for the upregulation of the retinoid receptors and possibly for the chemoprevention of lung carcinogenesis.

Table 1:

| Lung and Liver Samples (experiment B) | | | | |
|---|---|---|---|---|
| **Inhaled dose** | | **Inhalation Duration** | **Inhaled Dose (μg/kg) Dose (μg/kg)** | **Pulmonary Deposited** |
| 1 | (Vehicle Control) | 240 minutes | 0.0 | 0.0 |
| **2** | (Low) | 5 minutes 14 Days | 170 | 20 |
| **3** | (Low-Middle) | 15 minutes 14 Days | 500 | 50 |
| 4 | (Middle) | 45 minutes 14 Days | 1500 | 160 |
| 5 | (Middle-High) | 120 minutes 14 Days | 4000 | 440 |
| 6 | (High) | 240 minutes 14 Days | 8000 | 870 |
| Rats were killed between 2h and 50 min and 7h post exposure | | | | |

Table 2:

| TGase II Assay of Rat Lung Tissue (experiment B) | | | |
|---|---|---|---|
| Group | Protein assay | TGase II assay | |
| | ($\mu$g /$\mu$l) | DPM/$\mu$g protein | picomoles/$\mu$g protein/30 min |
| Vehicle Control | 31 | 3535 | $0.0450 \pm 0.003$ |
| Low | 31 | 7503 | $0.0955 \pm 0.004$ |
| Low-Middle | 32 | 9035 | $0.1150 \pm 0.006$ |
| Middle | 32 | 10449 | $0.1330 \pm 0\ 009$ |
| Middle-High | 33 | 8014 | $0.1020 \pm 0.005$ |
| High | 32 | 8053 | $0.1025 \pm 0.004$ |

Table 3:

| TGase II Assay of Rat Liver Tissue (experiment B) | | | |
|---|---|---|---|
| Group | Protein assay | TGase II assay | |
| | ($\mu$g /$\mu$l) | DPM/$\mu$g protein | picomoles/$\mu$g protein/30 min |
| Vehicle Control | 63 | 20426 | $0.260 \pm 0.005$ |
| Low | 61 | 22705 | $0.289 \pm 0.007$ |
| Low-Middle | 62 | 21448 | $0.273 \pm 0.018$ |
| Middle | 61 | 24590 | $0.313 \pm 0.025$ |
| Middle-High | 67 | 20348 | $0.269 \pm 0.015$ |
| High | 65 | 19719 | $0.271 \pm 0.015$ |

Table 4 :

| Rat Lung and Liver Samples (experiment A) | | | | |
|---|---|---|---|---|
| Exposure Group | Inhalation Duration | Projected Daily Inhaled | Projected Daily Total Deposited | Projected Daily Pulmonary Deposited |
| | | Dose (mg/kg) | Dose (mg/kg) | Dose (mg/kg) |
| 1 (Vehicle Control) | 2hr 1 Day | 0 | 0 | 0 |
| 2 (High) | 2 hr I Day | 6.6 | 2.0 | 0.7 |
| 3 (High) | 2 hr 17 Days | 6.6 | 2.0 | 0.7 |
| 4 (Vehicle Control) | 2hr 28 Days | 0 | 0 | 0 |
| 5 (Middle) | 2hr 28 Days | 2.0 | 0.6 | 0.2 |

Table 5:

| SENCAR Mice Liver Samples | | | |
|---|---|---|---|
| Group | # of Specimens | Experimental Period | Dietary |
| 3 | 5 | 75 Weeks | 3$\mu$g/g |
| 30 | 5 | 75 Weeks | 30$\mu$g/g |

Table 6 :

| TGase II Assay of SENCAR Mouse Liver Tissue | | | |
|---|---|---|---|
| Group | Protein assay | TGase II assay | |
| | (μg /μl) | DPM/μg protein | Picomoles/μg protein/30 minutes |
| 3 | 36 | 9,820 | 0.125 +/- 0.02 |
| 30 | 37 | 49,260 | 0.630 +/- 0.16 |

Table 7:

| SENCAR Mice Liver and Lung Samples | | | |
|---|---|---|---|
| Group | # of Specimens | Experimental Period | Dietary |
| 1 | 5 | 1 Day | 3μg/g |
| 2 | 5 | 1 Day | 30μg/g |
| 3 | 5 | 14 Days | 3μg/g |
| 4 | 5 | 14 Days | 30μg/g |
| 5 | 5 | 28 Days | 3μg/g |
| 6 | 5 | 28Days | 30μg/g |

## II. Methods for Upregulation Inhalation Experiments

### Animals and Treatment

Animals were housed individually in polycarbonate cages. General procedures for animal care and housing met current AAALAC standards, current requirements stated in the "Guide for Care and Use of Laboratory Animals" (National Academy of Sciences, 1996) and the U.S. Department of Agriculture through the Animal Welfare Act (Public Law 99-198).

Twelve hours of light and twelve hours of dark were provided in the animal rooms. A fluorescent light source was used, with lights turned on at ~0600 hours each day. The light/dark cycle was interrupted to allow for initiation or completion of study.

All study animals were introduced into the inhalation exposure tubes for at least 5 days with increasing duration up to 120 minutes prior to the first actual inhalation exposure. Within 24 hours of the last exposure, animals were euthanized by pentobarbital overdose; their lungs were removed, flash-frozen in liquid nitrogen and sent on dry ice to NCI for biomarker determination.

### Mice

Male A/J mice (Jackson Laboratories) 6-8 weeks old were quarantined a minimum of 7 days. Their diet throughout the experiment was AIN-76A. As part of a lung cancer chemoprevention study (Dahl et al., 2000) at 9-10 weeks of age, the mice were administered single 0.2 mL doses of a carcinogen solution (20 mg urethane in saline) by intraperitoneal injection. Daily 45-minute exposures to isotretinoin aerosol or vehicle were started the next day. Vehicle control mice were treated with the carcinogen and exposed to the aerosol vehicle; air control mice were treated with the carcinogen and maintained in cages without aerosol exposure. At first, exposure was daily for both doses, but after 12 days it was reduced to twice weekly for the higher dose because of severe local toxicity and thrice weekly for the lower dose as a precautionary measure.

### Rats: Study A

Male Sprague-Dawley rats were received from Charles River Laboratory. They were quarantined and observed for a period of seventy eight days prior to inhalation exposure to evaluate their health. Following an examination by a staff veterinarian, the animals were released for use on study. All animals were considered healthy and acceptable for use on the study. The rats were approximately 17 weeks of age and ranged in body weight from 512.2 to 6633 g on the first day of dose administration. The rats were allowed access to Certified Rodent Diet (P.M.I. Feeds, Inc.) *ad libitum* (except during dose administration). Fresh water from the Columbus Municipal Water supply was provided *ad libitum* (except during dose administration).

### Rats: Study B

Equal numbers of male and female Sprague-Dawley rats were received from Charles River Laboratory. They were quarantined and observed for a minimum period of seven days prior to inhalation exposure to evaluate their health. Following an examination by a staff veterinarian, the animals were released for use on study. All animals were considered healthy and acceptable for use in the study. The rats were 7-14 weeks of age and ranged in body weight from 200-350 g on the first day of exposure. They were allowed access to Certified Rodent Chow® 5002 (P.M.I. Feeds, Inc.) *ad libitum* (except during dose administration). Fresh water from the Columbus Municipal Water supply was provided *ad libitum* (except during dose administration).

### Isotretinoin

Isotretinoin (13-*cis*-Retinoic Acid) was received from Hande-Tech (Houston, TX) or Sigma-Aldrich (St. Louis, MO) of Toronto Research (North York, Ontario). The shipment was received at room temperature and was stored at ~5EC prior to formulation.

### Formulation of Nebulizer Solutions

### Mice

Powdered isotretinoin was dissolved in 100% ethanol plus 0.1% $\alpha$-tocopherol and 0.1% ascorbyl palmitate to give concentrations of isotretinoin ranging from 0.1 to 10 mg/ml. The formulations were prepared monthly. The solutions were protected from light and stored at -5°C until use. Ultraviolet-visible spectrophotometric verification of the formulated test article concentration was performed on all batches in advance of inhalation treatments with the test article solution. Only formulations within $\pm$ 10% of the targeted concentration were used on study.

### Rat Study A

Formulations of isotretinoin in 100% ethanol dosing solution were prepared at 1.4 mg/mL. Solutions were dispensed into amber glass bottles with Teflon®- lined lids and stored at ~5EC.

### Rat Study B

Powdered 13-*cis*-retinoic acid was dissolved in 10:90 (v/v) PEG 300:100% ethanol containing 0.5% (w/v) ascorbic acid and 0.5% (w/v) phosphatidyl choline. Sufficient test article was formulated for all treatment sessions. It was aliquoted into daily doses in amber vials and stored protected from light at ambient temperature. Verification of the concentration of the formulated test article was performed weekly on all batches. Only formulations with analysis results within $\pm$10% of the targeted concentration was used on study.

### Inhalation Exposure

Solutions were aerosolized using a Pari LC-plus nebulizer (Pari, Richmond, VA). Animals were exposed in nose-only exposure units designed to provide a fresh supply of the test atmosphere to each animal independent from the other animals. The exposure units were based on the design described by W. C. Cannon (Cannon *et al.,* 1983). The units consisted of multi-tier modular sections, each tier containing eight exposure ports located peripherally around a central delivery plenum.

During exposures, animals were restrained in unstoppered polycarbonate tubes (C&H Technologies, Westwood, NJ) through which a flow of aerosol, 350-500 mL/min per animal, passed from the chamber. The tubes were tapered on one end to approximately fit the shape of the animal's head and the diameter of the cylindrical portion of the cone was such that the animals could not turn in the cones. Each cone was fastened to the inhalation chamber with the nose portion of the cone protruding through a gasket into the chamber. This permitted the animal to breathe the test or control atmosphere emanating from within the control plenum. The flow rate through the chamber was set to provide approximately 350 to 500 mL/min for each animal. The exposure unit was operated under positive pressure.

*Aerosol Characterization*

To determine aerosol concentrations, measured volumes of aerosol were drawn through filters which subsequently were analyzed for isotretinoin by a UV/VIS method. To determine particle size, aerosol was drawn through Mercer-type cascade impactors (InTox, Albuquerque, NM) equipped with filters on each stage and a back up filter. The individual filters were analyzed for isotretinoin and the mass median aerodynamic diameters (MMADs) and geometric standard deviations (GSDs) were calculated from the data using Battelle software.

*Calculations of Deposited Dose*

Deposited doses were calculated as follows:

Aerosol concentration ($\mu$g / L) $\times$ (2.1 $\times$ BW(g)$^{0.75}$) mL / min $\times$ $\dfrac{1L}{1000\ mL}$ $\times$ Time (min) $\times$ $\dfrac{1}{BW\,(kg)}$ $\times$ $f$ where (2.1 $\times$ BW (g)$^{0.75}$) is the Guyton formula for minute volumes in mL/min (Guyton, 1947), BW is body weight and $f$ is the deposition fraction.

Fractional depositions were assumed the same as 1.09 and 1.03 $\mu$m monodisperse aerosols (Raabe *et al.*, 1988) for mice and rats, respectively.

*Aerosol Characteristics*

*Mice*

The mean aerosol concentrations and SDs were 1.3 $\pm$ 0.7 (SD) (N=12), 20.7 $\pm$ 10.1 (SD) (N=36) and 481 $\pm$ 234 (SD) (N=36) $\mu$g isotretinoin/L for the low, mid and high exposures, respectively. The MMADs and (GSDs) for the low, mid, and high doses were 1.00 (2.08), 1.33 (1.76), and 1.64 (2.61) $\mu$m, respectively.

*Rat Study A*

Aerosol characteristics are in Table 9.

*Rat Study B*

Targeted aerosol characteristics are in Table 10.

*Inhalation Exposures to Isotretinoin*

Ethanolic solutions of isotretinoin were aerosolized with particle sizes calculated to provide substantial pulmonary deposition. The vehicle vapors were not removed from the exposure air and may have had an effect on biomarkers, as the vehicle exposed animals had higher levels or some markers than unexposed controls. However, the effect was small and may have been influenced by the stress of handling and exposure. Stress has significant effects on some parameters, including tumorigenesis (Yamamoto *et al.*, 1995) and may have contributed to decreased tumor multiplicity in mice exposed to isotretinoin (Dahl *et al.*, 2000) and budesonide (Wattenberg *et al.*, 1997). In any case, the addition of isotretinoin to the aerosol at the mid dose level, produced a significant increase in biomarker expression relative to vehicle only aerosols.

Table 8.

| Dose of Inhaled Isotretinoin in Mice Exposed to Isotretinoin Aerosols | | | | |
|---|---|---|---|---|
| Mean Aerosol Concentration ($\mu$g/ L) | Calculated Average Daily Deposited Total Dose[a] | | Calculated Average Daily Deposited Pulmonary Dose[a] | |
| | Weeks 1-2 ($\mu$g/kg) | Weeks 3-10 ($\mu$g/kg) | Weeks 1-2 ($\mu$g/kg) | Weeks 3-10 ($\mu$g/kg) |
| 0[b] | 0 | 0 | 0 | 0 |
| 1.3 | 33.6 | 33.6 | 5.2 | 5.2 |
| 20.7 | 535 | 229 | 83 | 36 |

a. Vehicle control and low dose exposed 45 minutes daily throughout; high dose exposed 45 minutes daily during the first 12 days, 45 minutes 3 times per week, in Weeks 3-10.

b. Vehicle was 0.1% $\alpha$-tacopherol and 0.1% ascorbyl palmitate in ethanol.

Table 9.

| Dose of Inhaled Isotretinoin in Study A Rats Exposed to Isotretinoin Aerosols[a] | | |
|---|---|---|
| Daily Exposure Duration (min) | Calculated Total Daily Deposited Dose (µg/kg) | Calculated Pulmonary DailyDeposited Dose (µg/kg) |
| 240[b] | 0 | 0 |
| 5 | 39 | 15 |
| 15 | 117 | 44 |
| 45 | 351 | 131 |
| 120 | 936 | 350 |
| 240 | 1872 | 700 |

a. Four male rats for each exposure duration were exposed to isotretinoin aerosol at concentrations of ~62.2 µg/L, MMAD (GSD), 1.5 µm (~2.0) daily for 14 days and were sacrificed on Day 15.

b. Three vehicle control animals were exposed 240 minutes daily. The vehicle was 100% ethanol.

Table 10.

| Dose of Inhaled Isotretinoin in Study B Rats Exposed to Isotretinoin Aerosols[a] | | | |
|---|---|---|---|
| Study Duration (days) | Targeted Isotretinoin Aerosol Concentration (µg/ L) | Calculated Total Daily Deposited Dose (µg/kg) | Calculated Pulmonary Daily Deposited Dose (µg/kg) |
| 1[b] | 0 | 0 | 0 |
| 1 | 104 | 6436 | 2407 |
| 17 | 104 | 6436 | 2407 |
| 28[b] | 0 | 0 | 0 |
| 28 | 31 | 1918 | 718 |

a. Rats were exposed to aerosol 2 hours/day. Targeted MMADs (GSDs) were 1.3 µm (2.0).

b. Vehicle control rats were exposed for 1 or 28 days. Vehicle was 10:90 (%); PEG 300:100% ethanol, USP, 0.5% (w/v) ascorbic acid (w/v) 0.5% phosphotidyl choline.

### III. Demonstration That Inhaled Isotretinoin (13-*cis* retinoic acid) is an Effective Lung Cancer Chemopreventive Agent in A/J Mice at Low Doses.

The abbreviations used are: BaP for benzo(a)pyrene; NNK for 4-(methylnitrosamino)-1-(3-pyridyl)-1-butanone; RAR for retinoic acid receptor; SDS for sodium dodecylsulphate; PBS for phosphate buffered saline; EDTA for ethylenediaminetetraacetic acid; EGTA for ethylene glycol-bis[beta-aminoethyl ether]-N,N,N' N'-tetraacetic acid; PMSF for phenylmethylsulfonyl fluoride; HEPES for N-[2-hydroxyethyl]piperazine-N'-[2-ethanesulfonic acid]); DTT for dithiothreitol ; MMAD for mass median aerodynamic diameter; GSD for geometric standard deviation; IDV for integrated density value.

### Methods

#### *Lung Carcinogenesis Model*

The A/J mouse is a well-established animal model for preclinical chemoprevention studies. This strain has a hereditary predisposition for lung cancer, the so-called pulmonary adenoma susceptibility (Pas) genes. A strong candidate for one of these genes, Pas-1, is the K-*ras* protooncogene. Carcinogenesis in this model with NNK as the inciting agent has been studied so that the times required to develop hyperplastic areas, adenomas, and carcinomas are well known. In addition, the timing of molecular changes associated with carcinogenesis has been studied and are similar to those in humans. In both species, K-*ras* mutations are common early events.

#### *Experimental Design*

Mice were injected with one of three carcinogens and were exposed by inhalation in groups of 21 to three graded concentrations of isotretinoin or vehicle for 10 (urethane-treated mice) or 16 (NNK- and BaP-treated mice) weeks. Forty-six mice treated with each carcinogen and 46 untreated controls were maintained in cages and were not exposed. Some of these were sacrificed at intermediate times to determine the progress of carcinogenesis. At first, exposure was daily for all doses, but after 12 days it was reduced to twice weekly for the highest dose because of severe local toxicity and thrice weekly for the middle dose as a precautionary measure (Table 2).

### Animals and Treatment

Male A/J mice (Jackson Laboratories) 6-8 weeks old were quarantined a minimum of 7 days. Their diet throughout the experiment was AIN-76A, which, for NNK, gives higher tumor counts than NIH-07. At 9-10 weeks of age, the mice were administered single 0.2 ml doses (20 mg urethane in saline, 0.6 mg of NNK in saline, or 2 mg of BaP in tricaprylin) by intraperitoneal injection. Daily 45-min exposures to isotretinoin aerosol or vehicle were started the next day.

### Formulation of Nebulizer Solution

Powdered isotretinoin was dissolved in 100% ethanol plus 0.1% $\alpha$-tocopherol and 0.1% ascorbyl palmitate to give concentrations of isotretinoin ranging from 0.1 to 10 mg/ml. The formulations were prepared monthly. The solutions were protected from light and stored at -5°C until use. Ultraviolet-visible spectrophotometric verification of the formulated test article concentration was performed on all batches in advance of inhalation treatments with the test article solution. Only formulations within ± 10% of the targeted concentration were used on study.

### Inhalation Exposure

Solutions were aerosolized using a Pari LC-plus nebulizer (Pari, Richmond, VA). Animals were exposed in nose-only exposure units designed to provide a fresh supply of the test atmosphere to each animal, independent from other animals. The exposure units were based on the design described by W. C. Cannon (17). The units consisted of multi-tier modular sections, each tier containing eight exposure ports located peripherally around a central delivery plenum. During exposures, animals were restrained in unstoppered polycarbonate tubes (C&H Technologies, Westwood, NJ) through which a flow of aerosol, 350-500 ml/min per mouse, passed from the chamber. The tubes were tapered on one end to approximately fit the shape of the animal's head and the diameter of the cylindrical portion of the cone was such that the animals could not turn in the cones. Each cone was fastened to the inhalation chamber with the nose portion of the cone protruding through a gasket into the chamber, permitting the animal to breathe the test or control atmosphere emanating from within the central plenum.

### Aerosol Characterization

To determine aerosol concentrations, measured volumes of aerosol were drawn through filters which subsequently were analyzed for isotretinoin by a UV/VIS method. To determine particle size, aerosol was drawn through Mercer-type cascade impactors (InTox, Albuquerque, NM) equipped with filters on each stage and a back up filter. The individual filters were analyzed for isotretinoin and the mass median aerodynamic diameters (MMADs) and geometric standard deviations (GSDs) were calculated from the data using Battelle software.

### Quantitation of Lung Lesions

Within 24 h of the last inhalation exposure, animals were euthanized by intraperitoneal injection of pentobarbital and their lungs were removed and fixed in Bouin's solution or flash frozen for RAR determination. The lungs were evaluated in a blinded fashion so that neither carcinogen nor isotretinoin dose levels were known to the evaluator, who visually counted hyperplastic areas and adenomas on the lung pleural surface as previously described The significance of the differences between the mean tumor incidence of the treatment and the control groups was determined using the Mann-Whitney Rank Sum test (Statmost TM, DataMost Corp., Sandy, Utah).

### Biomarkers: RAR Induction

*Antibodies:* Polyclonal antibodies to RAR$\alpha$, $\beta$ and $\gamma$ (Santa Cruz Biotechnology Inc., San Francisco, CA) were used with a BM Chemiluminescence Western Blotting Kit (Mouse/Rabbit) (Boehringer Mannheim Corporation, Indianapolis).

*Apparatus and Reagents for Western Blot analysis:* X cell II Mini-cell & Blot Module was employed with 10% Tris-Glycine Gels and Transfer Buffer; Tris-Glycine SDS Sample Buffer; Tris-Glycine SDS was used as Running Buffer (NOVEX - NOVEL Experimental Technology Inc., San Francisco, CA).

*Experimental Design:* Five lungs each from the urethane-treated vehicle, low dose, and mid dose animals were snap frozen in liquid nitrogen and stored at -70°C for determination of RARs α, β and γ by Western Blot Analysis. In addition, five lungs each from urethane-injected unexposed mice, and untreated control mice were designated for Westerns. The lungs were homogenized and the RARs were determined by standard Western Blotting (21, 22). Due to deaths early in the high dose experiment, no lungs were available for Western Blot Analysis for the high dose exposures, i.e. all tissue was used for lesion quantitation purposes.

*Samples:* Lung tissue was collected, frozen on dry ice and kept at -70°C until used. A 500 mg portion was diced in small pieces and homogenized in 300 μl of cold PBS using a hand held homogenizer for 2-3 min. Samples were centrifuged at 500 rpm for 5 min or until the supernatant was clear. The pellet was suspended in 400 μl of cold Buffer A [2 μl 0.5 M EDTA, 10 μl 1 mM EGTA, 50 μl 100 mM PMSF, 10 μl 1M DTT, 10 ml (10 mM HEPES pH 7.9 + 10 mM KCl)], and left at ice temperature for 15 min. A 25 μl volume of 10% solution of NP-40 was added and the samples were mixed in a vortex vigorously for 10 sec. Samples were centrifuged at 14,000 rpm for 1 min at 4°C and the pellet was treated once more in this fashion. The supernatant was removed and 25 - 100 μl of Buffer C [4 μl 0.5 M EDTA, 20 μl 100 mM EGTA, 20 μl 0.1 M PMSF, 2 μl 1 M DTT, 2 ml (20 mM HEPES pH 7.9 + 0.4M NaCl)] was added. Pellets were resuspended by tapping gently on the bottom of the Eppendorf tube. Samples were rocked vigorously in a bucket of ice on an orbitor shaker for 30 min. Samples were centrifuged at 14,000 rpm for 5 min at 4°C. Supematants were kept frozen at -70°C until needed. Protein concentration determination was conducted by the Bradford method (23).

*Analysis:* Samples were prepared by adding one part of the Sample Buffer, to one part sample and mixing well. For denaturing conditions, samples were heated at 95°C for 5 min. Five μl of Rainbow Standard and 5 μl of Biotinylated Molecular Marker were used. Twenty μg protein per sample was loaded in each lane. Electrophoresis was performed with the voltage set at 125V for 1 - 1.5 h. Gel transfer was executed at 25V for 2 h. The membrane was stained in Ponceau S. for 5 min and destained with one wash of 5% acetic acid. The membrane was washed in TBS solution until the staining disappeared and then was incubated in 1 ml blocking solution and 9 ml TBS for 60 min along with 20 μl primary antibody solution overnight (dilution of 1:1000). The membrane was washed in PBS-Tween-20 three times for 10 min each after which it was incubated in 1 ml blocking solution and 19 ml TBS along with 20 μl Antibiotin HRP - Linked Antibody and 2 μl of secondary (rabbit anti-mouse antibody) (1:10,000 dilution) for 30 min. The membrane was washed in PBS-Tween-20 four times for 10 min each. The film was exposed to detect and develop.

### Aerosol Characteristics

The mean aerosol concentrations and SDs were $1.3 \pm 0.7$ (N=12), $20.7 \pm 10.1$ (N=36) and $481 \pm 234$ (N=36) μg isotretinoin/L for the low, mid and high exposures, respectively. The MMADs and (GSDs) for the low, mid, and high doses were 1.00 (2.08), 1.33 (1.76), and 1.64 (2.61) μm, respectively. [The progression to larger MMADs at the higher aerosol concentrations results from higher relative concentrations of non-volatiles, which dominate the compositions after the ethanol vehicle partially evaporates; thus, the percentages of non-volatiles-including α-tocopherol acetate and ascorbyl palmitate stabilizers-were 0.21, 0.3, and 1.2 leading to minimum droplet sizes (i.e., if all the ethanol had evaporated) of 0.38, 0.43, and 0.69 μm, respectively. The minimum droplet sizes were calculated by assuming an MMAD of 3 μm for the Pari-LC jet plus nebulizers used in these experiments and using the relationship $d_{final} = d_{orig} f$, where $d_{final}$ is the final diameter, $d_{orig}$ is the original diameter and f is the mass fraction of solute.]

### Calculations of Deposited Dose

Inhaled monodisperse particles having an aerodynamic diameter (AD) of 1.09 μm deposit 9.2% in the pulmonary region, with 59.2% total deposition (24). Taking the average mouse weight as 22 g, the respiratory minute volume—calculated as Raabe and coworkers (1998) had done— was $2.1 \times [\text{mass (g)}]^{0.75}$ ml/min (25). Assuming the same deposition efficiency as 1.09 μm monodispersed particles—for simplicity, as the actual values would vary somewhat for these aerosols—the calculated daily pulmonary doses of isotretinoin for each 45-min exposure were -0.005, 0.081 and 2 mg/kg per exposure. Calculated total deposited doses were 0.034, 0.54 and 12.4 mg/kg per exposure (Table 2).

### Chemoprevention by Inhaled Isotretinoin

All comparisons are to the vehicle-exposed control mice unless noted. Mice exposed to the high isotretinoin dose had substantial reductions in tumor multiplicity—ranging from 56% to 80% below vehicle controls—for all three carcinogens (Table 3) but during daily exposures for the first two weeks, experienced excessive toxicity to the snout and forelimbs. These mice lost weight (Fig. 1) and ~35% died. After a 2-day respite, exposure frequency was reduced to twice weekly, and the body weights increased to those of the vehicle exposed control mice (Fig. 1) and the lesions resolved, although two more mice died early in the study (Table 4). At the end of exposure, weights were again below those of the vehicle controls (Fig. 1). In light of the significant consequences of local retinoid toxicity in this model, extrapolation of these results to humans will be difficult.

NNK- and BaP-treated mice exposed to the mid isotretinoin-dose had reductions in multiplicity of tumor nodules by 88 and 67%, respectively (Table 3). At the end of exposure, the weights of these mice were 11 % below those of vehicle controls. Other signs of toxicity were absent, although 3% (2/63) of the mice died before the end of exposure (Table 4). [The reduction in body weight is of uncertain significance. Caloric restriction >10% for a significant portion of an animal's lifetime reduces tumorigenesis in some organs (26), but increases tumorigenesis in the lung (27).] Hyperplastic areas were significantly elevated in the mid and high isotretinoin-exposed, NNK- and BaP-treated mice. Total lesions, i.e., hyperplastic areas plus adenomas, were not affected by treatment in the NNK-induced animals, but were fewer for the urethane-treated animals at the high isotretinoin dose and for the BaP-treated animals at both the mid and high isotretinoin doses (Table 3).

For the low isotretinoin-dosed animals, the numbers of tumors were not affected by treatment at the 95% confidence level, but for both the NNK- and BaP-treated mice, trends in line with those of the mid and high isotretinoin-dosed mice were evident for both tumors and hyperplastic areas (Table 3).

For the urethane and BaP treatments, the mice exposed to vehicle had fewer tumors than the cage control animals (Table 3). This phenomenon was observed to an even greater degree in a chemoprevention study in A/J mice with aerosolized budesonide, where the control mice were exposed essentially to air only (28), and possibly is related to the tumorigenesis-inhibiting effect of stress (29), although a contribution from the ethanol vehicle or the antioxidant excipients, ascorbyl palmitate and $\alpha$-tocopherol, cannot be ruled out in the studies reported here.

### Biomarkers: RAR Induction

Inhaled isotretinoin upregulated lung tissue RAR a by 3.9 fold over solvent, RAR $\beta$ by 3.3 fold and RAR $\gamma$ by 3.7 fold (Figure 2 a and b). RARs might be useful biomarkers of inhaled isotretinoin activity, because all three genes contain retinoid response elements in their promoters and can be considered as first order responsive genes (30).

### DISCUSSION

Despite promising initial clinical reports and considerable basic interest in retinoids as lung cancer chemopreventive agents, there has been surprisingly limited work with these agents in preclinical efficacy models. In this pilot experiment, we used the carcinogen-induced A/J mouse model to begin to look at some simple pharmacology issues. The preliminary nature of this work precludes making definitive conclusions, but a series of observations are supportable.

### Inhalation Exposures to Isotretinoin

Ethanolic solutions of isotretinoin were aerosolized with particle sizes calculated to provide substantial pulmonary deposition. The ethanol was not removed from the exposure air. The inhaled ethanol, as well as the excipients, $\alpha$-tocopherol and ascorbyl palmitate, may have had an effect on carcinogenesis for the urethane and BaP treatments, as the vehicle exposed animals had fewer tumors than unexposed controls. However, the effect in these experiments—20 and 30% decreased tumor multiplicity for urethane and BaP, respectively—was less than that observed by others—50%—when BaP-treated control mice were exposed to, essentially, air alone. In any case, the addition of isotretinoin to the aerosols produced significant decreases in tumors relative to vehicle-only aerosols.

### Lung Tumor Prevention by Inhaled Retinoids

In this study, we looked at three different doses of isotretinoin aerosols inhaled daily. The lowest dose was not significantly effective. The highest dose was associated with lethal toxicity, presumably due to extensive ulceration of the snout and forearms of the mice. The assumption was that this was related to the well-known local toxic effects of retinoids on skin. This apparent local toxic response resolved with a reduction in dose frequency and significantly fewer lung nodules occurred for all three of the carcinogens with this dosing schedule. In light of the frequent lethal toxicity associated with the high dose exposures, however, we restricted our focus to the mid dose exposure as being the relevant drug dose.

With the mid dose, significant toxic signs were not observed other than the weight loss which occurred near the end of the study (Fig. 1). The three percent fatality rate in this cohort would not be unusual in an experiment involving this degree of manipulation of the experimental animals. Even in the vehicle controls there was a greater than 10% weight loss, relative to unexposed animals, due to the experimental procedure. The stress of forced aerosol inhalation in rodents is expected to be very different from voluntary aerosol inhalation in humans.

Despite the inhaled dose frequency being reduced as a precaution against potential local nasal toxicity, the mid dose was still associated with a significant reduction in the number of lung nodules for both of the tobacco-related carcinogens, BaP and NNK. This finding is even more significant when considering the amount of drug that was required to achieve this effect. For example, over most of the study the mid dose level, including extrapulmonary dose, was < 0.5% of an oral dose employed in the previously discussed in *vivo* experiments (Table 1); based on pulmonary dose alone (Table 2), the dosage was < 0.15% during the first two weeks and <0.06% during the remainder of the experiment. By all accounts, these comparisons suggest remarkable drug potency for the inhaled aerosol.

The finding that a modest dose of inhaled retinoid is both tolerated and efficacious supports the contention that lung therapy by inhalation is the preferred route of lung delivery for dealing with the airway-confined phase of a disease process as has been reported with certain agents used to treat pulmonary infections and corticosteroids for cancer prevention. An obvious application for the inhalation approach is the use of retinoids as lung cancer chemopreventive agents.

### *Hyperplasia and Total Lesions: Mode of Action*

The preliminary data for the NNK-treated mice suggest that isotretinoin does not eliminate initiated cells but inhibits their progression to the tumor stage since hyperplastic areas inversely correlated with tumors, while total lesions, i.e. hyperplastic areas plus adenomas, remained relatively constant (Table 3). A similar increase in hyperplastic areas occurred in the BaP-treated mice, but in this case, total lesions decreased, suggesting that initiated cells were either eliminated or were constrained to microscopic clusters.

BaP and NNK are putative major carcinogens in tobacco smoke, and thus are the most relevant of the carcinogens used in this study. The similarities between the dominant molecular lesions caused by BaP and NNK — BaP causes G-C to T-A transversions in the first nucleotide and NNK causes G-C to A-T transitions in the second nucleotide, both in codon 12 — argues against a substantial biological difference among cells initiated by the two carcinogens.

In contrast to the NNK- and BaP-treated animals, tumor multiplicity in the urethane-treated mice was decreased only at the high isotretinoin dose, the meaning of which is obfuscated by associated toxicity, and there was no effect on numbers of hyperplastic areas (Table 3). The total number of lesions was markedly reduced in the high dose animals, suggesting elimination of initiated cells or restriction of clonal expansion to microscopic lesions. Like NNK and BaP, urethane, an ethylating agent, mutates K-*ras,* but at codon 61 instead of codon 12. Morphological differences in tumors also occur. The fractions of tumors classified as solid tumors were 78% and 88% for BaP- and NNK-induced tumors, respectively, but only 57% for urethane-induced tumors. It is interesting to speculate that these differences contribute to the varied responses to inhaled isotretinoin, but there appears to be no supportive data in the literature.

### *Retinoid Toxicity at Efficacious Doses*

Although the high dose with the twice-weekly schedule was only 6% of a nontoxic oral dose (Table 1) it was associated with weight loss toward the end of the study (Fig. 1, Table 4). For the NNK- and BaP-treated mice, this dose was essentially no more efficacious than the much smaller mid dose (Table 3). Perhaps surprisingly, the mid inhaled dose at only 0.4% of a nontoxic oral dose also caused weight loss in mice exposed for >10 wks. Examination of the weight data over the duration of the experiment (data from BaP-treated mice in Fig 4, data from NNK- and urethane-treated mice not shown) confirms the late onset of the weight loss.

There are at least two possible explanations for this finding. First, the total dose may have been higher than calculated as a result of uptake through the skin of the exposed snout; second, local toxicity may have occurred in the respiratory tract. It seems unlikely that sufficient isotretinoin could have been absorbed through the skin to produce systemic toxicity nor would such a conclusion be supported by numerous inhalation studies in mice with aerosols of other compounds. This leaves local toxicity as a possible explanation. Microscopic examination of tissues for pathological changes was neither planned nor carried out for this pilot efficacy study; however, gross examination of the lungs revealed no differences between control and treated lungs except for the differences in numbers of tumors and hyperplastic areas. Given that the pulmonary dose is calculated to be only 13% of the total deposited dose and would be distributed over ~640 cm$^2$, pulmonary toxicity seems unlikely.

In contrast to the large surface of the lung, the upper respiratory tract, mostly nasal mucosa, has a surface area of only -3 cm$^2$ (37) but receives ~87% of the deposited dose. Coupling this high dose with the ease with which rodents develop debilitating nasal lesions we suggest that an explanation for the weight loss in the mid dose mice is local nasal toxicity, which developed to significant levels after ~10 wks of exposure.

Only toxicology studies with histopathology included will provide definitive explanations for the phenomenon of weight loss at these low doses, but if our suggestion that upper respiratory tract toxicity is to blame is correct, the observation is probably not relevant to the safety of inhaled retinoids in people. For pulmonary toxicology studies, except for neoplasia, nasal toxicity in rodents⸺which are obligate nose breathers⸺ from inhalants is usually not considered relevant for evaluating possible effects in humans unless human exposure will include the nasal cavity. In these exposure studies, due to the rodent physiology and anatomy, the administered drug dose to the nasopharynyx is many times higher for the rodents then would be expected in humans. For example, local nasal toxicity would not be a concern with a chemopreventive agent administered by oral inhalation since this route of administration skips drug transit across the nasal cavity. Moreover, the dose response curve for efficacy appears to have already plateaued at the mid dose (Table 3), suggesting that the dose could be lowered to a point between the low dose and the mid dose without sacrificing efficacy.

### *Limitations of the A/J Mouse Model: The Possible Role of Inflammation in Lung Cancer*

A special limitation of mouse inhalation models is that, with the nature of the drug delivery system, a major fraction of the total administered drug will deposit on the snout and in the upper respiratory tract Without delivering drug via a tracheostomy, there is no other alternative. Therefore, an artifact of this model is inefficient drug delivery to the deep lung. In humans, where much more efficient pulmonary drug delivery devices exist, the fraction of the drug that is impacted in and around the snout in the mouse would be expected to travel directly into the pulmonary airway. This improved drug delivery efficiency would greatly reduce the potential for local toxicity.

For some drugs, the high extrapulmonary deposition in the mouse model might confound interpretations regarding the effectiveness of the pulmonary route of drug delivery. In the case of isotretinoin, we suggest that the extrapulmonary deposited drug probably is not germane because it would either be swallowed or absorbed into the blood stream in much lower amounts than ineffective oral doses (Table 1) and so is unlikely to have contributed significantly to efficacy.

We used an animal model for evaluation of efficacy. Animal models for human lung cancer are widely accepted but, like all preclinical models, the A/J mouse model is imperfect. With the A/J model, mice treated with complete carcinogens do not develop lung inflammation and the attendant rapid cell proliferation that is common in human lung disease. The contribution of inflammation to aerodigestive cancerization is becoming more evident and this may be, in part, how retinoids may effect their chemopreventive benefit in humans.

A connection between the inflammation-associated enzyme, COX-2, and retinoid pathways is suggested by the fact that the Ras/ERK signaling pathway appears to play a role in the regulation of COX-2 expression. Human non-small cell lung cancer (NSCLC) cell lines with mutations in K-*ras* have high expression levels of COX-2, and inhibition of *ras* activity in these cell lines decreases COX-2 expression. Rat intestinal epithelial cells and fibroblasts transfected with H-*ras* overexpress COX-2, whereas inhibitors of ERK ameliorate this response. We and others have found COX activity to be potentially significant in aerodigestive cancers. A high percentage of murine and human lung adenocarcinomas have a mutated ras gene and a constitutively activated ras signaling pathway which may explain the high levels of COX-2 seen in some lung tumors. RAR-β is known to interfere with the *ras* signaling pathway by inhibiting the function of the AP-1 transcription factor. An expected result of this interference would be the down-regulation of COX-2 expression, which may play a role in the decreased tumorigenesis seen in the A/J lung cancer model following isotretinoin inhalation. Such an effect of RARs on COX-2 expression is supported by published data showing that retinoids inhibit the EGF (i.e., *ras*)-induced transcription of COX-2 in human oral squamous carcinoma cells.

Through time, the development of *in vivo* models that more closely mirror the actual process of carcinogenesis in humans would be highly desirable. For the pilot evaluations discussed in this manuscript, we think the A/J mouse model is adequate as long as its shortcomings are acknowledged. In future experiments, the local aerosol drug dose in and on the snout can be reduced through modifications of the exposure system to prevent facial exposure and by reducing the particle diameter to -0.3 μm, which will increase the pulmonary to total dose ratio to ~64% (24).

### *Induction of RARs*

RARs were investigated as biomarkers because their genes contain retinoic acid response elements and as such are likely to be upregulated soon after exposure to retinoids, i.e. they are first order dependence genes (30). The induction of all three RARs was at least three fold in lungs exposed to mid levels of isotretinoin. Only the urethane-treated mice were examined in this pilot study, but these probably represent the other treatment groups for this determination as all mice were exposed to the same aerosols. The induction of the RARs in the mid dose mice correlated with efficacy in the BaP- and NNK-treated mice and may not only provide biomarkers for exposure, but may also be a part of the mechanism for the efficacy of inhaled isotretinoin. The upregulation of lung RARs by inhaled isotretinoin occurs across species as reported in a companion paper. This paper also indicates that oral administration induces liver, but not lung RARs, and that administration by inhalation induces only lung, and not liver receptors.

### *Implications of Improving the Therapeutic Index of Retinoid Administration*

The clinical trials to unequivocally establish the chemopreventive benefit of oral isotretinoin are likely to be completed in the near future. Even if positive, however, long-term compliance is expected to be a major issue due to the significant frequency of debilitating side effects. Even if changing the route of administration of retinoids only decreased the side effect profile, this would make the drug much more interesting to contemplate for broad clinical utility. Moreover, because the role of retinoids in maintaining optimal bronchial epithelial differentiation has been extensively studied, other general beneficial effects ofretinoic acids on epithelia have been well documented. For example, using a rodent model of chronic obstructive pulmonary disease (COPD), there is a suggestion that retinoids can reverse parenchymal lung injuries associated with compromised respiratory function. Indeed, if there is a causal relationship, this benefit may contribute to the cancer preventive effects since individuals suffering from COPD and other smoking related diseases are at increased risk for developing lung cancer.

### *Conclusion*

In this preliminary analysis of the pulmonary delivery of isotretinoin by inhalation, there was evidence of efficacy at weekly pulmonary doses as low as 0.25 mg/kg and suggested efficacy even at 0.04 mg/kg in reducing the pulmonary carcinogenicity of the tobacco carcinogens, NNK and BaP, in A/J mice. Since pulmonary drug delivery deposits drug directly on the tumor compartment, efficacy can be achieved at low doses: mid and low weekly pulmonary doses were < 2% and < 0.3%, respectively, of the highest recommended weekly oral dose of isotretinoin for acne treatment. The results reported here, however, are all the more encouraging as they probably were caused by the <10% of the inhaled aerosol that deposited in the lung, as the extrapulmonary dose was probably too low to have a systemic effect. This suggests that an improved therapeutic index can be achieved in humans by more selectively delivering retinoid chemopreventive agents to deep lung tissue using aerosols. Further work with this approach, both preclinically and in the clinic, are justified to validate the true benefit of this important new chemoprevention delivery approach.

Table 1

| Comparison of Oral versus Inhalation Route for Lung Tumor Chemopreventive Efficacy of Isotretinoin in A/J Mice | | |
|---|---|---|
| Weekly Ingested or Deposited Inhaled Dose (Route) (mg/kg) | Duration (Weeks) | Efficacy (Carcinogen) |
| 200 (po)[a] | 20 | No (urethane) |
| 400 (po)[a] | 20 | No (urethane) |
| 0.24 (inh)[b] | 10-16 | Maybe[c] (NNK, BaP) No (urethane) |
| 1.6 (inh)[b] | 10-16 | Yes (NNK, BaP) No (urethane) |
| 24.9 (inh)[b] | 10-16 | Yes (urethane, NNK, BaP) |

a. Frasca and Garfinkel, 1981. Assuming 4 g of feed ingested daily per 21 gm mouse.

b. Total calculated deposited dose (Table 2).

c. Trend for efficacy: p<0.13 for NNK-treated; p<0.30 for BaP-treated.

*Table 2* Isotretinoin Doses in Pilot Efficacy Studies

| Exposure Level | Mean Aerosol Concentration (μg/L) | Pulmonary Deposited Isotretinoin Dose/ Exposure Day[a] (μg/kg) | Total Deposited Isotretinoin Dose/ Exposure Day[b] (μg/kg) | Weekly Pulmonary Dose | | Weekly Total Dose | |
|---|---|---|---|---|---|---|---|
| | | | | Weeks 1-2 (mg/kg) | Weeks 3-16 (mg/kg) | Weeks 1-2 (mg/kg) | Weeks 3-16 (mg/kg) |
| Low[c] | 1.3 | 5.2 | 33.6 | 0.037 | 0.037 | 0.235 | 0.235 |
| Mid[c] | 20.7 | 83 | 535 | 0.582 | 0.25 | 3.75 | 1.60 |
| High[c] | 481 | 1931 | 2400 | 13.5 | 3.86 | 87.0 | 24.9 |

a.  Aerosol concentration $(\mu g/L) \times (2.1 \times 22^{0.75})$ mL/min $\times$ 1 L/1000 mL $\times$ 45 min $\times$ 1/0.022 kg $\times$ 0.092: where $(2.1 \times 22^{0.75})$ is the Guyton formula for min volumes, in mL/min, of a 22 gm mouse (25) and 0.092 is the pulmonary deposition fraction of a 1.09 μm (AD) monodisperse aerosol (24). Note: Reported minute volumes for mice range from slightly less than the Guyton value used here to slightly greater than 2-fold (50), but as Raabe and coworkers (1988) based fraction of deposition on the Guyton formula, it is appropriate to use it here as well.

b.  Calculated by substituting total deposition fraction (0.592) (24) in place of the pulmonary deposition (0.092) in footnote a.

c.  Low exposed daily throughout; mid and high exposed daily during the first 12 days, 3x- and 2x/week, respectively, in Weeks 3-16.

EP 1 263 420 B1

EP 1 263 420 B1

*Table 3* Lung Tumorigenesis Inhibition in A/J Mice

| Carcinogen | Weekly 13-*cis* Pulmonary Dose[a] (mg/kg) | Tumors Per Lung Set (Mean ± SE) | Hyperplastic Areas Per Lung Set (Mean ± SE) | Total Lesions Per Lung Set (Mean ± SE) |
|---|---|---|---|---|
| Urethane | Cage Control | $29.9 \pm 1.5$ | $1.5 \pm 0.3$ | $31.4 \pm 1.6$ |
| | Vehicle Control | $24.2 \pm 1.9$ | $4.6 \pm 0.9$ | $29.2 \pm 2.2$ |
| | 0.04 | $25.7 \pm 1.7$ | $3.6 \pm 1.1$ | $29.3 \pm 1.5$ |
| | 0.25 | $26.4 \pm 2.1$ | $5.9 \pm 1.2$ | $32.2 \pm 2.4$ |
| | 4.0 | $10.6 \pm 1.2^{d}$ | $5.3 \pm 1.3$ | $15.9 \pm 1.9^{d}$ |
| NNK | Cage Control | $1.9 \pm 0.3$ | $4.5 \pm 0.5$ | $6.5 \pm 0.5$ |
| | Vehicle Control | $2.5 \pm 0.4$ | $1.3 \pm 0.3$ | $3.8 \pm 0.5$ |
| | 0.04 | $2.1 \pm 0.3^{e}$ | $2.0 \pm 0.4$ | $4.1 \pm 0.6$ |
| | 0.25 | $0.3 \pm 0.2^{d}$ | $3.3 \pm 0.3^{d}$ | $3.6 \pm 0.3$ |
| | 4.0 | $0.8 \pm 0.3^{d}$ | $3.8 \pm 0.6^{c}$ | $4.5 \pm 0.7$ |
| BaP | Cage Control | $12.8 \pm 1.1$ | $1.9 \pm 0.4$ | $14.7 \pm 1.1$ |
| | Vehicle Control | $9.0 \pm 1.0$ | $0.2 \pm 0.1$ | $9.2 \pm 0.9$ |
| | 0.04 | $6.4 \pm 0.7^{f}$ | $0.8 \pm 0.3$ | $7.2 \pm 0.8$ |
| | 0.25 | $3.0 \pm 0.5^{d}$ | $3.2 \pm 0.4^{d}$ | $6.1 \pm 0.5^{b}$ |
| | 4.0 | $1.8 \pm 0.5^{d}$ | $3.3 \pm 0.9^{d}$ | $5.2 \pm 1.0^{b}$ |

a. Rounded from Table 2 values for Weeks 3-16
b. $p < 0.05$ compared to vehicle control
c. $p < 0.005$ compared to vehicle control
d. $p < 0.0005$ compared to vehicle control
e. Trend for fewer tumors, $p < 0.30$ compared to vehicle control
f. Trend for fewer tumors, $p < 0.13$ compared to vehicle control

*Table 4* Body Weights of Carcinogen-Treated, Isotretinoin-Exposed *A/J* Mice Near Termination of Exposures (gms) (Mean ± SD) (N)

| Isotretinoin Aerosol Level | Urethane-Treated Mice Day 60 | NNK-Treated Mice Day 102 | BaP-Treated Mice Day 102 |
|---|---|---|---|
| Unexposed Control | 24.7 ± 2.1 (45) | 26.6 ± 2.2 (41) | 25.6 ± 2.6 (41) |
| Vehicle Control | 20.8 ± 1.2 (21) | 22.7 ± 1.0 (21) | 22.5 ± 1.4 (21) |
| Low Dose | 21.0 ± 1.2 (21) | 22.1 ± 1.5 (21) | 21.8 ± 1.2 (21) |
| Mid Dose | 20.7 ± 1.5 (21) | 20.3 ± 1.2 (20)[a,b] | 20.1 ± 1.8 (20)[a,b] |
| High Dose | 18.9 ± 1.7 (12)[a,c] | 17.5 ± 1.2 (14)[a,d] | 19.3 ± 1.6 (12)[a,e] |

a. $p < 0.05$, different from vehicle control.
b. One NNK-treated mouse sacrificed moribund in Wk 13; one BaP-treated mouse found dead in Wk 4.
c. Nine mice found dead or sacrificed moribund in Wk 2.
d. Five mice found dead or sacrificed moribund in Wk 2; 1 in Wk 5; 1 in Wk 7.
e. Nine mice found dead or sacrificed moribund in Wk 2.

### *Summary of Isotretinoin Experiments*

In previously treated head-and-neck cancer patients, orally administered isotretinoin (13-*cis* retinoic acid) reduced the occurrence of second aerodigestive tumors, including lung tumors, but side effects made chronic therapy problematic. We reasoned that inhaled isotretinoin might provide sufficient drug to the target cells for efficacy while avoiding systemic toxicity, and we proceeded with the pilot study reported here. Male A/J mice were given single intraperitoneal (IP) doses of urethane, a common experimental lung carcinogen, or benzo(a)pyrene (BaP) or 4-(methylnitrosamino)-1-(3-pyridyl)-1-butanone (NNK), putative major carcinogens in tobacco smoke. The next day, exposures to isotretinoin aerosols for 45 min daily at 1.3, 20.7 or 481 µg/L were started. After two weeks, the high dose caused severe toxicity, necessitating a reduction of dose frequency to twice a week. As a precaution, the mid dose was reduced to three exposures per week. The weekly total deposited doses after the dose frequency reductions were calculated to be 0.24, 1.6 and 24.9 mg/kg for the low, mid and high doses, of which 16% was estimated to be deposited in the lungs. The weekly deposited pulmonary drug doses were calculated to be 0.01, 0.07 and 1.1% of a previously reported ineffective oral dose in urethane-treated A/J mice. After 10-16 weeks, mice were sacrificed to count areas of pulmonary hyperplasia and adenomas. For all carcinogens, the mice exposed to the high isotretinoin dose showed reductions of tumor multiplicity ranging from 56 to 80% (p<0.005). The mid dose was associated with reductions of tumor multiplicity by 67 and 88% (p<0.005) in BaP- and NNK- treated mice, respectively, and was tolerated until ~12 wks when the mice began losing weight. The low dose mice had non-significant reductions of 30% (p<0.13) and 16% (p<0.30) for BaP and NNK treated mice, respectively without any evidence of side effects. For BaP- and NNK-treated mice, numbers of hyperplastic areas directly correlated to dose level and inversely to tumor number, suggesting arrested progression. Inhaled mid dose isotretinoin caused upregulation of lung tissue nuclear retinoic acid receptors relative to vehicle-exposed mice, RARα ( 3.9-fold vehicle), RARβ (3.3-fold) and RARγ (3.7-fold), suggesting that these receptors may be useful biomarkers of retinoid activity in this system. The encouraging results from this pilot study suggest that inhaled isotretinoin merits evaluation in people at high risk for lung cancer.

## IV. Electrohydrodynamic Aerosols

Therapeutic formulations must be compatible with an aerosol-generating device so that an aerosol cloud with certain preferred characteristics can be reproduced each time the device is used. Aerosols having uniform particles are desirable over aerosols with nonuniform particles because of the improved deposition characteristics of the aerosol. Used with a compatible formula, electrohydrodynamic aerosol generating devices are capable of creating monomodal aerosols having particles more uniform in size than with other devices or methods.

Typically, electrohydrodynamic devices include a spray nozzle in fluid communication with a source of liquid to be aerosolized, at least one discharge electrode, a first power first voltage source for maintaining the spray nozzle at a negative (or positive) potential relative to the potential of the discharge electrode, and a second voltage source for maintaining the discharge electrode at a positive (or negative) potential relative to the potential of the spray nozzle.

An electrohydrodynamic device creates an aerosol by causing a liquid to form droplets that enter a region of high electric field strength. The electric field imparts a net electric charge to these droplets of liquid, and this net electric charge tends to remain on the surface of the droplet. The repelling force of the charge on the surface of the droplet balances against the surface tension of the liquid in the droplet, thereby causing the droplet to form a cone-like structure known as a Taylor Cone. In the tip of this cone-like structure, the electric force exerted on the surface of the droplet overcomes the surface tension of the liquid, thereby generating a stream of liquid that disperses into a many smaller droplets of roughly the same size. These smaller droplets form a mist which forms the aerosol cloud that the user ultimately inhales.

In the use of the present invention the medicament is to be delivered to the patient or test subject using an electrohydrodynamic aerosol generating device. The use of an electrohydrodynamic aerosol generating device achieves greater drug efficacy because the drug is delivered directly to the tissues or organ (e.g., epithelial tissues, lungs, etc.) requiring treatment thereby reducing the total dosage or amount of drug that must be delivered to the recipient. Controlled particle size and predictable deposition patterns make an electrohydrodynamic aerosol generating devices superior to other aerosol generating devices for applications such as those represented by the present invention. Reduced dosages and targeted delivery also serve to reduce or minimize undesired exposure of adjacent tissues to anticancer drugs as well as reducing or minimizing systemic toxicity. In a preferred embodiment of the present invention, the particle size of the aerosol cloud generated by an electrohydrodynamic aerosol generating device is about 1.0 to 6.0 micrometers. Please see pending U.S. provisional patent application 60/132,215 "Therapeutic Formulations for Aerosolization and Inhalation". Also see pending U.S. patent applications 09/263,986 "Pulmonary Dosing System and Method" and 09/220,249 "Pulmonary Aerosol Delivery Device and Method".

### Example

### Aerosolized 13-cis Retinoic Acid

Pulmonary Dose Range

5.2 µg/kg daily
83 µg/kg 3 x per week
1931 µg/kg 2 x per week

mice 5-2000 µg/kg body weight deposited daily pulmonary dose
mice 0.03-0.17-67.6 ng/cm$^2$ lung surface area
human 0.03-0.17-67.6 ng/cm$^2$ lung surface area
human 3-1310 µg/kg body weight for equivalent lung dose

While the above description contains many specificities, these should not be construed as limitations on the scope of the invention, but rather as exemplification of preferred embodiments. Numerous other variations of the present invention are possible, and it is not intended herein to mention all of the possible equivalent forms or ramifications of this invention. Various changes may be made to the present invention without departing from the scope of the invention.

**Claims**

1. Use of retinoic acid or a retinoic acid derivative as a chemoprotectant in the manufacture of a medicament administered on a chronic basis via inhalation in an effective amount for inhibiting progression of neoplasms of the aerodigestive tract in a patient at risk for developing lung cancer from such progression said medicament being adapted for administration by an electrohydrodynamic device, wherein said retinoic acid or retinoic acid derivative is administered at from 0.03 to 0.17 ng/cm$^2$ lung surface area or at 0.84 to 230 µg/g of human lung tissue

2. The use according to Claim 1 wherein said effective amount of inhaled retinoic acid or retinoic acid derivative activates retinoic acid receptors in the lung of such patient.

3. The use according to Claim 2 wherein said retinoic acid or retinoic acid derivative is selected from the group consisting of 13-cis retinoic acid, all-trans retinoic acid, 9-cis retinoic acid, 11-cis retinoic acid, and retinol.

4. The use according to Claim 2 wherein said retinoic acid or retinoic acid derivative is selected from the group consisting of 13-cis retinoic acid and all-trans retinoic acid.

5. The use according to Claim 1 wherein said retinoic acid or retinoic acid derivative is administered at from 0.03 to 0.05 ng/cm$^2$ lung surface area.

6. The use according to Claim 1 wherein said retinoic acid or retinoic acid derivative is administered to said patient once per day.

7. The use according to Claim 1 wherein said patient has been diagnosed with lung cancer and treated for inhibition or removal of such lung cancer prior to initiation of chronic treatment by inhalation of such retinoic acid or retinoic acid derivative.

8. The use according to Claim 7 wherein the patient undergoes treatment by surgery, radiation or chemotherapy or a combination of these treatment regimens prior to initiation of inhalation therapy with said retinoic acid or retinoic acid derivative.

9. The use according to either of Claim 7 or Claim 8 wherein said retinoic acid or retinoic acid derivative is administered at from 0.03 to 0.17 ng/cm$^2$ lung surface area.

10. The use according to Claim 9 wherein said retinoic acid or retinoic acid derivative is administered at from 0.03 to 0.05 ng/cm$^2$ lung surface area.

11. The use according to Claim 1 wherein said inhaled retinoic acid or retinoic acid derivative is administered using an electrohydrodynamic device and wherein the respirable particles of said inhaled retinoic acid derivative are in the size range of from 0.5 to 6.0 micrometers.

12. The use according to Claim 2 wherein said retinoic acid or retinoic acid derivative is administered to a patient at a dose of from 0.03 to 0.17 ng/cm$^2$ lung surface 3 times per week.

**EP 1 263 420 B1**

**Patentansprüche**

1. Verwendung von Retinoesäure oder einem Retinoesäure-Derivat als Chemoprotektans bei der Herstellung eines Medikaments, das auf chronischer Basis über Inhalation in einer wirksamen Menge zur Inhibierung der Progression von Neoplasmen des Aerodigestionstrakts bei einem Patienten verabreicht wird, der einem Risiko für die Entwicklung von Lungenkrebs aus einer derartigen Progression unterliegt, wobei das Medikament zur Verabreichung durch eine elektrohydrodynamische Vorrichtung angepasst ist, wobei die Retinoesäure oder das Retinoesäure-Derivat mit 0,03 bis 0,17 ng/cm$^2$ Lungenoberfläche oder mit 0,84 bis 230 µg/g Humanlungengewebe verabreicht wird.

2. Verwendung nach Anspruch 1, bei der die wirksame Menge an inhalierter Retinoesäure oder inhaliertem Retinoesäure-Derivat Retinoesäure-Rezeptoren in der Lunge eines solchen Patienten aktiviert.

3. Verwendung nach Anspruch 2, bei der die Retinoesäure oder das Retinoesäure-Derivat ausgewählt ist aus der Gruppe bestehend aus 13-cis-Retinoesäure, all-trans-Retinoesäure, 9-cis-Retinoesäure, 11-cis-Retinoesäure und Retinol.

4. Verwendung nach Anspruch 2, bei der die Retinoesäure oder das Retinoesäure-Derivat ausgewählt ist aus der Gruppe bestehend aus 13-cis-Retinoesäure und all-trans-Retinoesäure.

5. Verwendung nach Anspruch 1, bei der die Retinoesäure oder das Retinoesäure-Derivat mit 0,03 bis 0,05 ng/cm$^2$ Lungenoberfläche verabreicht wird.

6. Verwendung nach Anspruch 1, bei der die Retinoesäure oder das Retinoesäure-Derivat dem Patienten einmal täglich verabreicht wird.

7. Verwendung nach Anspruch 1, bei der bei dem Patienten Lungenkrebs diagnostiziert worden ist und der vor dem Beginn der chronischen Behandlung durch Inhalation der Retinoesäure oder des Retinoesäure-Derivats bezüglich der Inhibierung oder der Entfernung des Lungenkrebses behandelt worden ist.

8. Verwendung nach Anspruch 7, bei der sich der Patient vor Beginn der Inhalationstherapie mit der Retinoesäure oder dem Retinoesäure-Derivat einer Behandlung durch Operation, Bestrahlung oder Chemotherapie oder einer Kombination dieser Behandlungsschemata unterzieht.

9. Verwendung nach Anspruch 7 oder Anspruch 8, bei der die Retinoesäure oder das Retinoesäure-Derivat mit 0,03 bis 0,17 ng/cm$^2$ Lungenoberfläche verabreicht wird.

10. Verwendung nach Anspruch 9, bei der die Retinoesäure oder das Retinoesäure-Derivat mit 0,03 bis 0,05 ng/cm$^2$ Lungenoberfläche verabreicht wird.

11. Verwendung nach Anspruch 1, bei der die inhalierte Retinoesäure oder das inhalierte Retinoesäure-Derivat unter Verwendung einer elektrohydrodynamischen Vorrichtung verabreicht wird und bei der die einatembaren Teilchen des inhalierten Retinoesäure-Derivats im Größenbereich von 0,5 bis 6,0 Mikrometer liegen.

12. Verwendung nach Anspruch 2, bei der die Retinoesäure oder das Retinoesäure-Derivat einem Patienten mit einer Dosis von 0,03 bis 0,17 ng/cm$^2$ Lungenoberfläche 3 mal pro Woche verabreicht wird.

**Revendications**

1. Utilisation d'acide rétinoïque ou d'un dérivé d'acide rétinoïque en tant que chimioprotecteur dans la fabrication d'un médicament, administré de manière chronique par inhalation en une quantité efficace pour inhiber la progression de néoplasmes du tractus aérodigestif chez un patient à risque de développer un cancer du poumon à partir d'une telle progression, ledit médicament étant adapté à l'administration par un dispositif électrohydrodynamique, dans laquelle ledit acide rétinoïque ou dérivé d'acide rétinoïque est administré à hauteur de 0,03 à 0,17 ng/cm$^2$ d'aire surfacique de poumon ou à hauteur de 0,84 à 230 µg/g de tissu pulmonaire humain.

2. Utilisation selon la revendication 1, dans laquelle ladite quantité efficace d'acide rétinoïque ou de dérivé d'acide rétinoïque inhalé active les récepteurs d'acide rétinoïque dans le poumon d'un tel patient.

27

**3.** Utilisation selon la revendication 2, dans laquelle ledit acide rétinoïque ou dérivé d'acide rétinoïque est choisi dans le groupe consistant en l'acide 13-cis-rétinoïque, l'acide tout-trans-rétinoïque, l'acide 9-cis-rétinoïque, l'acide 11-cis-rétinoïque et le rétinol.

**4.** Utilisation selon la revendication 2, dans laquelle ledit acide rétinoïque ou dérivé d'acide rétinoïque est choisi dans le groupe consistant en l'acide 13-cis-rétinoïque et l'acide tout-trans-rétinoïque.

**5.** Utilisation selon la revendication 1, dans laquelle ledit acide rétinoïque ou dérivé d'acide rétinoïque est administré à hauteur de 0,03 à 0,05 ng/cm$^2$ d'aire surfacique de poumon.

**6.** Utilisation selon la revendication 1, dans laquelle ledit acide rétinoïque ou dérivé d'acide rétinoïque est administré audit patient une fois par jour.

**7.** Utilisation selon la revendication 1, dans laquelle ledit patient a été diagnostiqué d'un cancer du poumon et traité pour l'inhibition ou l'élimination d'un tel cancer du poumon avant l'amorçage du traitement chronique par inhalation d'un tel acide rétinoïque ou dérivé d'acide rétinoïque.

**8.** Utilisation selon la revendication 7, dans laquelle le patient subit un traitement par chirurgie, radiothérapie ou chimiothérapie ou une combinaison de ces régimes de traitement avant l'amorçage de la thérapie par inhalation avec ledit acide rétinoïque ou dérivé d'acide rétinoique.

**9.** Utilisation selon la revendication 7 ou la revendication 8, dans laquelle ledit acide rétinoïque ou dérivé d'acide rétinoïque est administré à hauteur de 0,03 à 0,17 ng/cm$^2$ d'aire surfacique de poumon.

**10.** Utilisation selon la revendication 9, dans laquelle ledit acide rétinoïque ou dérivé d'acide rétinoïque est administré à hauteur de 0,03 à 0,05 ng/cm$^2$ d'aire surfacique de poumon.

**11.** Utilisation selon la revendication 1, dans laquelle ledit acide rétinoïque ou dérivé d'acide rétinoïque inhalé est administré en utilisant un dispositif électrohydrodynamique et dans laquelle les particules respirables dudit dérivé d'acide rétinoïque inhalé sont dans la plage de taille allant de 0,5 à 6,0 micromètres.

**12.** Utilisation selon la revendication 2, dans laquelle ledit acide rétinoïque ou dérivé d'acide rétinoïque est administré à un patient en une dose allant de 0,03 à 0,17 ng/cm$^2$ de surface pulmonaire trois fois par semaine.

**Figure 1A**

EP 1 263 420 B1

## Figure 1B

Figure 1C

Figure 1D

# Figure 2A

1. Vehicle Control 1 Day
2. High dose 13-*cis* RA 1 Day
3. High dose 13-*cis* RA 17 Day
4. Vehicle Control 28 Day
5. Middle dose 13-*cis* RA 28 Day

EP 1 263 420 B1

Figure 2B

## Figure 3A

1. Vehicle Control
2. Low dose 13-*cis* RA
3. Low-Middle dose 13-*cis* RA
4. Middle dose 13-*cis* RA
5. Middle-High dose 13-*cis* RA
6. High dose 13-*cis* RA

Figure3B

# Figure 4A

3. Liver from SENCAR mice fed a physiological RA diet (3μg/g) for 75 weeks
30. Liver from SENCAR mice fed a pharmacological RA diet (30μg/g) for 75 weeks

Figure 4B

EP 1 263 420 B1

Figure 4C

# Figure 5A

Day 1    Day 14    Day 28
3    30    3    30    3    30    KDa

RARα → ← 57
← 46.5

RARβ → ← 57
← 46.5

RARγ → ← 57
← 46.5

MW

3. Liver from SENCAR mice fed a physiological RA diet (3µg/g) for 1, 14 and 28 days
30. Liver from SENCAR mice fed a pharmacological RA diet (30µg/g) for 1, 14 and 28 days

Figure 5B

**Figure 6. Body Weights of BaP-Treated A/J Mice Exposed to Isotretinoin Aerosols.** The body weights for the BaP-treated mice are representative and typical of those for all three carcinogen treatments.

   a. Daily exposures.
   b. Daily exposures first twelve days, then thrice weekly.
   c. Daily exposures first twelve days, then twice weekly.

**Figure 7. RAR Induction Determinations in A/J Mice Exposed to Isotretinoin Aerosols.**
Fifteen male A/J mice were divided into 5 experimental groups and given single intraperitoneal doses of urethane (UR) or no treatment. Group 1: 3 mice given UR then inhaled air; Group 2: 3 mice given UR then inhaled vehicle aerosol; Group 3: 3 mice given UR then inhaled low isotretinoin aerosol concentration; Group 4: 3 mice given UR then inhaled mid isotretinoin aerosol concentration; Group 5: 3 mice were not treated.
   a. Western blot analysis of lung tissue; details given under Materials and Methods.
   b. Densitometric analysis of western blots in Fig 2a. IDV = integrated density values.